(19) Europäisches Patentamt
European Patent Office
Office européen des brevets

(11) EP 4 464 706 A2

(12) EUROPEAN PATENT APPLICATION

(43) Date of publication:
**20.11.2024 Bulletin 2024/47**

(21) Application number: **24172671.0**

(22) Date of filing: **26.04.2024**

(51) International Patent Classification (IPC):
***C07D 471/04*** *(2006.01)* ***H10K 30/30*** *(2023.01)*
***H10K 39/32*** *(2023.01)* ***H10K 85/60*** *(2023.01)*

(52) Cooperative Patent Classification (CPC):
**C07D 471/04; H10K 30/30; H10K 39/32; H10K 85/60**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **27.04.2023 KR 20230055798**

(71) Applicant: **Samsung Electronics Co., Ltd.**
**Suwon-si, Gyeonggi-do 16677 (KR)**

(72) Inventors:
- **YI, Jeoung In**
  **16678 Suwon-si (KR)**
- **KIM, Hyeong-Ju**
  **16678 Suwon-si (KR)**
- **MINAMI, Daiki**
  **16678 Suwon-si (KR)**
- **PARK, Kyung Bae**
  **16678 Suwon-si (KR)**
- **PARK, Jeong Il**
  **16678 Suwon-si (KR)**
- **YUN, Sungyoung**
  **16678 Suwon-si (KR)**
- **CHOI, Tae Jin**
  **16678 Suwon-si (KR)**
- **HEO, Chul Joon**
  **16678 Suwon-si (KR)**

(74) Representative: **Elkington and Fife LLP**
**Prospect House**
**8 Pembroke Road**
**Sevenoaks, Kent TN13 1XR (GB)**

(54) **2,7-BIS(PHENYL)-BENZO[LMN][3,8]PHENANTHROLINE-1,3,6,8(2H,7H)-TETRONE DERIVATIVES FOR USE AS MATERIALS IN PHOTOELECTRONIC DEVICES AND IMAGE SENSORS**

(57) A compound is represented by Chemical Formula 1, and at least one of $X^1$ and $X^2$ is a substituent having a volume ranging from about 900 $bohr^3$ to about 3000 $bohr^3$ (V/molecule), and a photoelectric device, an image sensor, and an electronic device includes the compound:

[Chemical Formula 1]

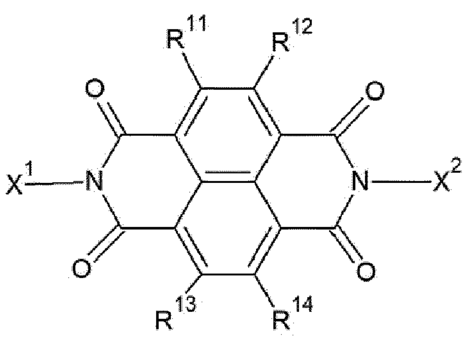

In Chemical Formula 1, each substituent is the same as defined in the detailed description.

FIG. 1

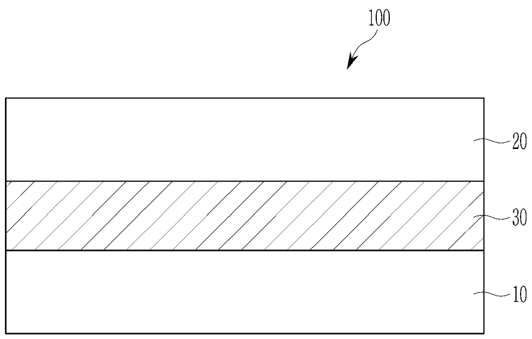


EP 4 464 706 A2

## Description

### FIELD OF THE INVENTION

Example embodiments relate to compounds, photoelectric devices, image sensors, and electronic devices.

### BACKGROUND OF THE INVENTION

A photoelectric device may convert light into an electrical signal using photoelectric effects. A photoelectric device may include a photodiode, a phototransistor, etc., and may be applied to an image sensor, etc.

An image sensor including a photodiode may require high resolution and thus a small pixel. At present, a silicon photodiode is widely used. In some cases, a silicon photodiode exhibits a problem of deteriorated sensitivity because of a relatively small absorption area due to relatively small pixels. Accordingly, an organic material that is capable of replacing silicon has been researched.

An organic material may have a relatively high extinction coefficient and may selectively absorb light in a particular wavelength region depending on a molecular structure, and thus may simultaneously replace a photodiode (e.g., a silicon photodiode) and a color filter and resultantly improve sensitivity and contribute to relatively high integration.

Therefore, interest in organic materials that can be used in these sensors is increasing.

### SUMMARY OF THE INVENTION

Some example embodiments provide a compound that can significantly improve the photoelectric conversion efficiency of a device and has little change in efficiency depending on a deposition rate.

Some example embodiments also provide a photoelectric device having excellent photoelectric conversion efficiency including the above compound.

Some example embodiments also provide an image sensor including the photoelectric device.

Some example embodiments also provide an electronic device including the image sensor.

According to some example embodiments, a compound which is represented by Chemical Formula 1 may be provided.

[Chemical Formula 1]

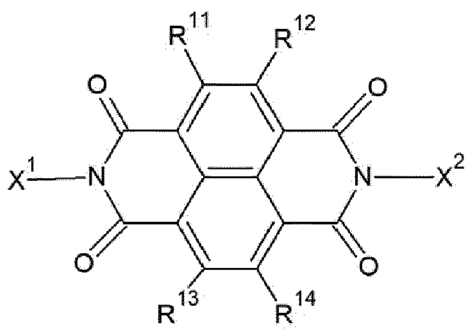

In Chemical Formula 1,

$X^1$ and $X^2$ are each independently hydrogen, deuterium, tritium, a substituted or unsubstituted C1 to C10 alkyl group, a substituted or unsubstituted C1 to C10 alkoxy group, a substituted or unsubstituted C6 to C30 aryl group, or a substituted or unsubstituted C3 to C30 heteroaryl group,

at least one of $X^1$ or $X^2$ is a substituent having a volume of about 900 bohr$^3$ to about 3000 bohr$^3$ (V/molecule), and the at least one of $X^1$ or $X^2$ is a substituted or unsubstituted C6 to C30 aryl group or a substituted or unsubstituted C3 to C30 heteroaryl group, and

$R^{11}$, $R^{12}$, $R^{13}$, and $R^{14}$ are each independently hydrogen, deuterium, tritium, a halogen, a cyano group, a C1 to C10 alkyl group, a C1 to C10 alkoxy group, or a C6 to C10 aryl group.

The compound may have an energy disorder of less than or equal to about 0.22 eV.

The compound may have a volume of about 3800 bohr$^3$ to about 7000 bohr$^3$ (V/molecule).

The compound may have a volume ratio of substituents obtained according to Equation 1 and Equation 2,

ranging from about 45% to about 85%.

[Equation 1]

$$\text{Volume } (V_b) \text{ of substituent} = V_a - V_{Ref}$$

[Equation 2]

$$\text{Volume ratio of a substituent} = V_b/V_a$$

In Equations 1 and 2,
$V_a$ is a volume of the compound, $V_b$ is a volume of the substituents ($X^1$ and $X^2$), and $V_{Ref}$ is a volume of the compound represented by Chemical Formula Ref.

[Chemical Formula Ref]

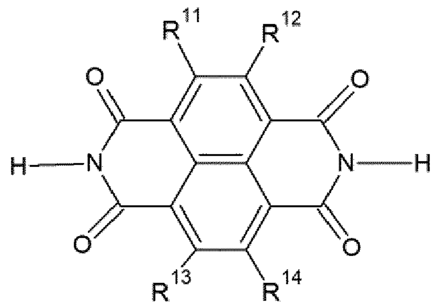

In Chemical Formula Ref, $R^{11}$, $R^{12}$, $R^{13}$, and $R^{14}$ are the same as in Chemical Formula 1.In Chemical Formula 1, at least one of $X^1$ and $X^2$ may include an electron donating group.

The electron donating group may be a C3 to C20 branched alkyl group, a C3 to C20 branched alkoxy group, a C6 to C20 aryl group, a C3 to C20 cycloalkyl group, a C3 to C20 heteroaryl group, a C3 to C20 heterocycloalkyl group, or any combination thereof.

In Chemical Formula 1, at least one of $X^1$ and $X^2$ may include $-Si(R^a)(R^b)(R^c)$ (wherein $R^a$, $R^b$, and $R^c$ may each independently be hydrogen, deuterium, tritium, a halogen, a cyano group, a linear or branched C1 to C10 alkyl group, or a C6 to C10 aryl group), $-Ge(R^a)(R^b)(R^c)$ (wherein $R^a$, $R^b$, and $R^c$ are each independently hydrogen, deuterium, tritium, a halogen, a cyano group, a linear or branched C1 to C10 alkyl group, or a C6 to C10 aryl group), or any combination thereof. In Chemical Formula 1, about 10% to about 100% of the hydrogen atoms of at least one of $X^1$ and $X^2$ may be substituted with deuterium or tritium based on a total number of hydrogen atoms present in each substituent.

At least one of $X^1$ or $X^2$ may be a substituent selected from Group 1.

[Group 1]

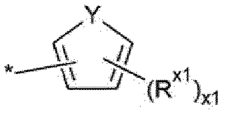
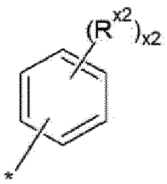
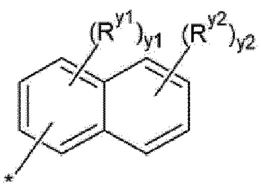
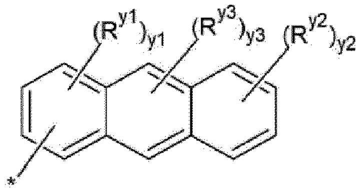
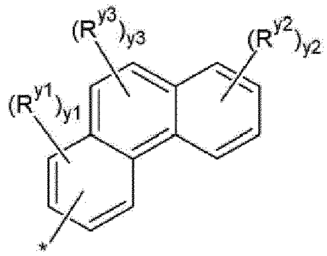
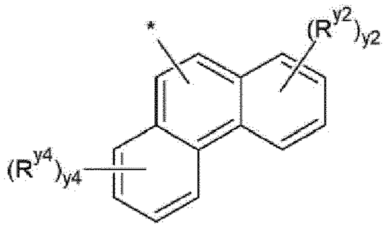
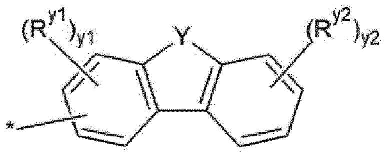

In Group 1,

Y is S, O, Se, Te, or CRR' (wherein R and R' are each independently hydrogen, deuterium, tritium, a C1 to C10 alkyl group, a C1 to C10 alkoxy group, or a C6 to C10 aryl group),

$R^{x1}$ is deuterium, tritium, a C3 to C20 branched alkyl group, a C3 to C20 branched alkoxy group, a C6 to C20 aryl group, a C3 to C20 cycloalkyl group, a C3 to C20 heteroaryl group, a C3 to C20 heterocycloalkyl group, -Si($R^a$)($R^b$)($R^c$) (wherein $R^a$, $R^b$, and $R^c$ are each independently hydrogen, deuterium, tritium, a halogen, a cyano group, a linear or branched C1 to C10 alkyl group, or a C6 to C10 aryl group), - Ge($R^a$)($R^b$)($R^c$) (wherein $R^a$, $R^b$, and $R^c$ are each independently hydrogen, deuterium, tritium, a halogen, a cyano group, a linear or branched C1 to C10 alkyl group, or a C6 to C10 aryl group), or any combination thereof,

x1 is an integer of 1 to 3,

$R^{x2}$ is deuterium, tritium, a C3 to C20 branched alkyl group, a C3 to C20 branched alkoxy group, a C6 to C20 aryl group, a C3 to C20 cycloalkyl group, a C3 to C20 heteroaryl group, a C3 to C20 heterocycloalkyl group, -Si($R^a$)($R^b$)($R^c$) (wherein $R^a$, $R^b$, and $R^c$ are each independently hydrogen, deuterium, tritium, a halogen, a cyano group, a linear or branched C1 to C10 alkyl group, or a C6 to C10 aryl group), - Ge($R^a$)($R^b$)($R^c$) (wherein $R^a$, $R^b$, and $R^c$ are each independently hydrogen, deuterium, tritium, a halogen, a cyano group, a linear or branched C1 to C10 alkyl group, or a C6 to C10 aryl group), or any combination thereof,

x2 is an integer of 1 to 5,

$R^{y1}$, $R^{y2}$, $R^{y3}$, and $R^{y4}$ are each independently hydrogen, deuterium, tritium, halogen, a cyano group, C1 to C10 linear alkyl group, C1 to C10 linear alkoxy group, C3 to C20 branched alkyl group, C3 to C20 branched alkoxy group, C6 to C20 aryl group, C3 to C20 cycloalkyl group, C3 to C20 heteroaryl group, C3 to C20 heterocycloalkyl group, -Si($R^a$)($R^b$)($R^c$) (wherein $R^a$, $R^b$, and $R^c$ are each independently hydrogen, deuterium, tritium, a halogen, a cyano group, a linear or branched C1 to C10 alkyl group, or a C6 to C10 aryl group), -Ge($R^a$)($R^b$)($R^c$) (wherein $R^a$, $R^b$, and $R^c$ are each independently hydrogen, deuterium, tritium, a halogen, a cyano group, a linear or branched C1 to C10 alkyl group, or a C6 to C10 aryl group), or any combination thereof,

y1 is an integer of 0 to 3,

y2 is an integer of 0 to 4,

y3 is an integer of 0 to 2,

y4 is an integer of 0 to 4, and

* is a linking point with Chemical Formula 1.

In Group 1, $R^{y1}$, $R^{y2}$, $R^{y3}$, and $R^{y4}$ may each independently be deuterium, tritium, halogen, a cyano group, C1 to C10 linear alkyl group, C1 to C10 linear alkoxy group, C3 to C20 branched alkyl group, C3 to C20 branched alkoxy group, C6 to C20 aryl group, C3 to C20 cycloalkyl group, C3 to C20 heteroaryl group, C3 to C20 heterocycloalkyl group, -Si($R^a$)($R^b$)($R^c$) (wherein $R^a$, $R^b$, and $R^c$ are each independently hydrogen, deuterium, tritium, a halogen, a cyano group, a linear or branched C1 to C10 alkyl group, or a C6 to C10 aryl group), -Ge($R^a$)($R^b$)($R^c$) (wherein $R^a$, $R^b$, and $R^c$ are each independently hydrogen, deuterium, tritium, a halogen, a cyano group, a linear or branched C1 to C10 alkyl group, or a C6 to C10 aryl group), or any combination thereof.

The compound represented by Chemical Formula 1 may have a reorganization energy of less than or equal to about 0.390 eV.

The compound represented by Chemical Formula 1 may have an oscillator strength of greater than or equal to about 0.40.

The compound represented by Chemical Formula 1 may have a molecular weight of less than or equal to about 1500 g/mol.

The compound may have a sublimation temperature of less than or equal to about 340 °C (wherein the sublimation temperature refers to a temperature at which a 10% weight loss of the compound occurs compared to an initial weight of the compound when thermogravimetrically analyzed at 10 Pa or less).

According to some example embodiments, a photoelectric device (e.g., organic photoelectric device) may include a first electrode and a second electrode facing each other, and

an active layer between the first electrode and the second electrode,
wherein the active layer may include the compound represented by Chemical Formula 1.

According to some example embodiments, an image sensor including the photoelectric device is provided.

In some example embodiments, the image sensor may include a semiconductor substrate integrated with a plurality of first photo-sensing devices configured to sense light in a blue wavelength region and a plurality of second photo-sensing devices configured to sense light in a red wavelength region, and the photoelectric device may be on the semiconductor substrate and configured to selectively sense light in a green wavelength region.

The active layer may include a p-type semiconductor and an n-type semiconductor, and the n-type semiconductor may include the compound represented by Chemical Formula 1.

In some example embodiments, the image sensor may further include a color filter layer including a blue filter configured to selectively transmit light in a blue wavelength region and a red filter configured to selectively transmit light in a red wavelength region.

In some example embodiments, the first photo-sensing device and the second photo-sensing device may be stacked in a vertical direction in a semiconductor substrate.

In some example embodiments, the image sensor may include a green photoelectric device that is configured to selectively sense light in a green wavelength region and is the photoelectric device, a blue photoelectric device configured to selectively sense light in a blue wavelength region, and a red photoelectric device configured to selectively sense light in a red wavelength region, which may be stacked.

In some example embodiments, the image sensor may include a green photoelectric device that is configured to selectively sense light in a green wavelength region and is the photoelectric device, a blue photoelectric device configured to selectively sense light in a blue wavelength region, and a red photoelectric device configured to selectively sense light in a red wavelength region, which are aligned in parallel on the semiconductor substrate and overlap in a horizontal direction that is parallel to an upper surface of the semiconductor substrate.

According to some example embodiments, an electronic device including the photoelectric device or image sensor is provided.

The compound can significantly improve the photoelectric conversion efficiency of the device and has excellent deposition stability since there is little change in efficiency depending on the deposition rate, thereby improving processability when manufacturing the device.

## BRIEF DESCRIPTION OF THE DRAWINGS

FIG. 1 is a cross-sectional view showing a photoelectric device according to some example embodiments,
FIG. 2 is a cross-sectional view showing a photoelectric device according to some example embodiments,
FIG. 3 is a schematic plan view showing an organic CMOS image sensor according to some example embodiments,
FIG. 4 is a cross-sectional view of the organic CMOS image sensor of FIG. 3,
FIG. 5 is a schematic cross-sectional view of an organic CMOS image sensor according to some example embodiments,
FIG. 6 is a schematic cross-sectional view of an organic CMOS image sensor according to some example embodiments,
FIG. 7 is a cross-sectional view of an organic CMOS image sensor according to some example embodiments,
FIG. 8 is a schematic view showing an organic CMOS image sensor according to some example embodiments,
FIG. 9 is a cross-sectional view of the organic CMOS image sensor of FIG. 8,
FIG. 10 is a perspective view schematically showing an organic CMOS image sensor according to some example

embodiments,

FIG. 11 schematically shows a view of cross-section of an example of the organic CMOS image sensor of FIG. 10 taken along line XI-XI' of FIG. 10,

FIG. 12 is a block diagram of a digital camera including an image sensor according to some example embodiments, and

FIG. 13 is a schematic diagram of an electronic device according to some example embodiments.

## DETAILED DESCRIPTION OF THE EMBODIMENTS

Hereinafter, some example embodiments are described in detail so that those of ordinary skill in the art can easily implement them. However, a structure that is actually applied may be implemented in various different forms, and is not limited to the example embodiments described herein.

In the drawings, the thickness of layers, films, panels, regions, etc., are exaggerated for clarity. Like reference numerals designate like elements throughout the specification. It will be understood that when an element such as a layer, film, region, or substrate is referred to as being "on" another element, it can be directly on the other element or intervening elements may also be present. In contrast, when an element is referred to as being "directly on" another element, there are no intervening elements present.

In the drawings, parts having no relationship with the description are omitted for clarity of some example embodiments, and the same or similar constituent elements are indicated by the same reference numeral throughout the specification.

Hereinafter, the terms "lower portion" and "'upper portion" are used for better understanding and ease of description, but do not limit the position relationship.

As used herein, "at least one of A, B, or C," "one of A, B, C, or any combination thereof" and "one of A, B, C, and any combination thereof" refer to each constituent element, and any combination thereof (e.g., A; B; C; A and B; A and C; B and C; or A, B and C).

As used herein, when specific definition is not otherwise provided, "substituted" refers to replacement of a hydrogen of a compound or a functional group by a halogen atom (F, Br, Cl, or I), a hydroxy group, a nitro group, a cyano group, an azido group, an amidino group, an amine group (-NR'R", wherein R' and R" are independently a hydrogen atom, a C1 to C20 alkyl group or a C6 to C30 aryl group), a hydrazino group, a hydrazono group, a carbonyl group, a carbamyl group, a thiol group, an ester group, a carboxyl group or a salt thereof, sulfonic acid group or a salt thereof, a phosphoric acid group or a salt thereof, a C1 to C20 alkyl group, a C1 to C20 alkoxy group, a C2 to C20 alkenyl group, a C2 to C20 alkynyl group, a C6 to C30 aryl group, a C7 to C30 arylalkyl group, a C2 to C20 heteroaryl group, a C3 to C20 heteroarylalkyl group, a C3 to C30 cycloalkyl group, a C3 to C15 cycloalkenyl group, a C6 to C15 cycloalkynyl group, a C2 to C20 heterocycloalkyl group, -Si($R^a$)($R^b$)($R^c$) (wherein $R^a$, $R^b$, and $R^c$ are each independently hydrogen, deuterium, tritium, a halogen, a cyano group, a linear or branched C1 to C10 alkyl group, or a C6 to C10 aryl group), -Ge($R^a$)($R^b$)($R^c$) (wherein $R^a$, $R^b$, and $R^c$ are each independently hydrogen, deuterium, tritium, a halogen, a cyano group, a linear or branched C1 to C10 alkyl group, or a C6 to C10 aryl group), or any combination thereof.

As used herein, "aryl group" refers to a cyclic functional group wherein all elements of the cycle (all ring-forming atoms) have p-orbitals which form conjugation, and may be a monocyclic, polycyclic, or fused ring polycyclic (e.g., rings sharing adjacent pairs of carbon atoms) functional group.

As used herein, "arene group" refers to a hydrocarbon cyclic group having an aromatic ring, and includes monocyclic and multicyclic hydrocarbon cyclic groups, and additional rings of the multicyclic hydrocarbon cyclic group may be an aromatic ring or a non-aromatic ring. The arene group may be a C6 to C30 arene group, a C6 to C20 arene group, or a C6 to C10 arene group.

As used herein, "heteroarene group" refers to an arene group including 1 to 3 heteroatoms selected from N, O, S, P, and Si in the ring. The heteroarene group may be a C3 to C30 heteroarene group, a C3 to C20 heteroarene group, or a C3 to C10 heteroarene group.

As used herein, "hydrocarbon cyclic group" may be a C3 to C30 hydrocarbon cyclic group. The hydrocarbon cyclic group may be an aromatic hydrocarbon cyclic group (e.g., C6 to C30 arene group, C6 to C20 arene group, or C6 to C10 arene group or C6 to C30 aryl group, C6 to C20 aryl group, or C6 to C10 aryl group), an alicyclic hydrocarbon cyclic group (e.g., C3 to C30 cycloalkyl group, C5 to C30 cycloalkyl group, C3 to C20 cycloalkyl group, or C3 to C10 cycloalkyl group), or a fused ring group thereof. For example, the fused ring group may refer to a fused ring of an aromatic ring (arene ring) and a non-aromatic ring (alicyclic ring), for example a fused ring in which at least one aromatic ring (arene ring) such as a C6 to C30 arene group, a C6 to C20 arene group, or a C6 to C10 arene group or a C6 to C30 aryl group, a C6 to C20 aryl group, or a C6 to C10 aryl group and at least one non-aromatic ring (alicyclic ring) such as a C3 to C30 cycloalkyl group, a C3 to C20 cycloalkyl group, or a C3 to C10 cycloalkyl group are fused to each other.

As used herein, "cycloalkyl group" refers to a monovalent saturated hydrocarbon cyclic group in which the atoms of the cycle are carbon, for example a cyclopropyl group, a cyclobutyl group, a cyclopentyl group, or a cyclohexyl

group.

As used herein, when a definition is not otherwise provided, "aromatic ring" refers to a C6 to C10 hydrocarbon cyclic group (e.g., a C6 to C10 aryl group) providing a conjugated structure or a C2 to C10 heterocyclic group (e.g., a C2 to C10 heteroaryl group) providing a conjugated structure.

As used herein, when a definition is not otherwise provided, "spiro structure" may be a substituted or unsubstituted C5 to C30 hydrocarbon cyclic group, a substituted or unsubstituted C2 to C30 heterocyclic group, or a fused ring thereof. The substituted or unsubstituted C5 to C30 hydrocarbon cyclic group may be for example a substituted or unsubstituted C5 to C30 cycloalkyl group (e.g., a substituted or unsubstituted C5 to C20 cycloalkyl group or a substituted or unsubstituted C5 to C10 cycloalkyl group) or a substituted or unsubstituted C6 to C30 aryl group (e.g., a substituted or unsubstituted C6 to C20 aryl group, or a substituted or unsubstituted C6 to C10 aryl group) and the substituted or unsubstituted C2 to C30 heterocyclic group may be for example a substituted or unsubstituted C2 to C20 heterocycloalkyl group (e.g., a substituted or unsubstituted C2 to C10 heterocycloalkyl group) or a substituted or unsubstituted C2 to C20 heteroaryl group (e.g., a substituted or unsubstituted C2 to C10 heteroaryl group).

As used herein, when a definition is not otherwise provided, "fused ring" is a fused ring of two or more substituted or unsubstituted C5 to C30 hydrocarbon cyclic groups, a fused ring of two or more substituted or unsubstituted C2 to C30 heterocyclic groups, or a fused ring of a substituted or unsubstituted C5 to C30 hydrocarbon cyclic group and a substituted or unsubstituted C2 to C30 heterocyclic group (e.g., a fluorenyl group). Herein, the hydrocarbon cyclic group and the hetero cyclic group are as defined above.

As used herein, when specific definition is not otherwise provided, "'hetero" refers to one including 1 to 3 heteroatoms selected from N, O, S, P, and Si.

As used herein, "heteroaryl group" refers to an aryl group containing 1 to 3 heteroatoms selected from N, O, S, P, and Si in the ring. The heteroaryl group may be a C3 to C30 heteroaryl group, a C3 to C20 heteroaryl group, or a C3 to C10 heteroaryl group.

As used herein, "alkyl group" refers to a monovalent linear or branched saturated hydrocarbon group, for example a methyl group, an ethyl group, a propyl group, an isopropyl group, an n-butyl group, an isobutyl group, a t-butyl group, a pentyl group, a hexyl group, and the like.

As used herein, when a definition is not otherwise provided, "alkoxy group" is expressed as -OR, where R may be the alkyl group described above.

As used herein, when a definition is not otherwise provided, "combination" means a mixture, laminate, or alloy of constituents.

As used herein, when a definition is not otherwise provided, "combination" in the definition of a substituent refers to substitution in which one substituent is substituted with another substituent, fusion with each other, or a linkage to each other by a single bond or a C1 to C10 alkylene group.

It will further be understood that when an element is referred to as being "on" another element, it may be above or beneath or adjacent (e.g., horizontally adjacent) to the other element. It will be understood that elements and/or properties thereof (e.g., structures, surfaces, directions, or the like), which may be referred to as being "perpendicular," "parallel," "coplanar," or the like with regard to other elements and/or properties thereof (e.g., structures, surfaces, directions, or the like) may be "perpendicular," "parallel," "coplanar," or the like or may be "substantially perpendicular," "substantially parallel," "substantially coplanar," respectively, with regard to the other elements and/or properties thereof. Elements and/or properties thereof (e.g., structures, surfaces, directions, or the like) that are "substantially perpendicular" with regard to other elements and/or properties thereof will be understood to be "perpendicular" with regard to the other elements and/or properties thereof within manufacturing tolerances and/or material tolerances and/or have a deviation in magnitude and/or angle from "perpendicular," or the like with regard to the other elements and/or properties thereof that is equal to or less than 10% (e.g., a. tolerance of ±10%). Elements and/or properties thereof (e.g., structures, surfaces, directions, or the like) that are "substantially parallel" with regard to other elements and/or properties thereof will be understood to be "parallel" with regard to the other elements and/or properties thereof within manufacturing tolerances and/or material tolerances and/or have a deviation in magnitude and/or angle from "parallel," or the like with regard to the other elements and/or properties thereof that is equal to or less than 10% (e.g., a. tolerance of ±10%). Elements and/or properties thereof (e.g., structures, surfaces, directions, or the like) that are "substantially coplanar" with regard to other elements and/or properties thereof will be understood to be "coplanar" with regard to the other elements and/or properties thereof within manufacturing tolerances and/or material tolerances and/or have a deviation in magnitude and/or angle from "coplanar," or the like with regard to the other elements and/or properties thereof that is equal to or less than 10% (e.g., a. tolerance of ±10%). It will be understood that elements and/or properties thereof may be recited herein as being "identical" to, "the same" or "equal" as other elements and/or properties, and it will be further understood that elements and/or properties thereof recited herein as being "identical" to, "the same" as, or "equal" to other elements and/or properties may be "identical" to, "the same" as, or "equal" to or "substantially identical" to, "substantially the same" as or "substantially equal" to the other elements and/or properties thereof. Elements and/or properties thereof that are "substantially identical" to, "substantially the same" as or "substantially equal" to other elements

and/or properties thereof will be understood to include elements and/or properties thereof that are identical to, the same as, or equal to the other elements and/or properties thereof within manufacturing tolerances and/or material tolerances. Elements and/or properties thereof that are identical or substantially identical to and/or the same or substantially the same as other elements and/or properties thereof may be structurally the same or substantially the same, functionally the same or substantially the same, and/or compositionally the same or substantially the same. While the term "same," "equal" or "identical" may be used in description of some example embodiments, it should be understood that some imprecisions may exist. Thus, when one element or value is referred to as being the same as another element or value, it should be understood that an element or a value is the same as another element or value within a desired manufacturing or operational tolerance range (e.g., ±10%). It will be understood that elements and/or properties thereof described herein as being the "substantially" the same and/or identical encompasses elements and/or properties thereof that have a relative difference in magnitude that is equal to or less than 10%. Further, regardless of whether elements and/or properties thereof are modified as "substantially," it will be understood that these elements and/or properties thereof should be construed as including a manufacturing or operational tolerance (e.g., ±10%) around the stated elements and/or properties thereof. When the terms "about" or "substantially" are used in this specification in connection with a numerical value, it is intended that the associated numerical value include a tolerance of ±10% around the stated numerical value. Moreover, when the words "about" and "substantially" are used in connection with geometric shapes, it is intended that precision of the geometric shape is not required but that latitude for the shape is within the scope of the inventive concepts. Further, regardless of whether numerical values or shapes are modified as "about" or "substantially," it will be understood that these values and shapes should be construed as including a manufacturing or operational tolerance (e.g., ±10%) around the stated numerical values or shapes. When ranges are specified, the range includes all values therebetween such as increments of 0.1%.

As used herein, when a definition is not otherwise provided, an energy level is a highest occupied molecular orbital (HOMO) energy level or a lowest unoccupied molecular orbital (LUMO) energy level.

As used herein, when a definition is not otherwise provided, a work function or an energy level is expressed as an absolute value from a vacuum level. In addition, when the work function or the energy level is referred to be deep, high, or large, it may have a large absolute value based on "0 eV" of the vacuum level while when the work function or the energy level is referred to be shallow, low, or small, it may have a small absolute value based on "0 eV" of the vacuum level. Also, the difference between the work function and/or the energy level may be a value obtained by subtracting a small absolute value from a large absolute value.

As used herein, when a definition is not otherwise provided, the HOMO energy level may be evaluated by an amount of photoelectrons emitted according to energy by irradiating UV light on a thin film using AC-2 (Hitachi) or AC-3 (Riken Keiki Co., LTD.).

As used herein, when a definition is not otherwise provided, the LUMO energy level is obtained by measuring the energy bandgap using a UV-Vis spectrometer (Shimadzu Corporation), and then calculating the LUMO energy level from the energy bandgap and the already measured HOMO energy level.

As used herein, a "volume of the substituent" may be obtained by calculating a volume of the compound under the b3lyp/6-31G condition using Gaussian 09 and subtracting a volume of the compound represented by Chemical Formula Ref from the calculated volume.

[Chemical Formula Ref]

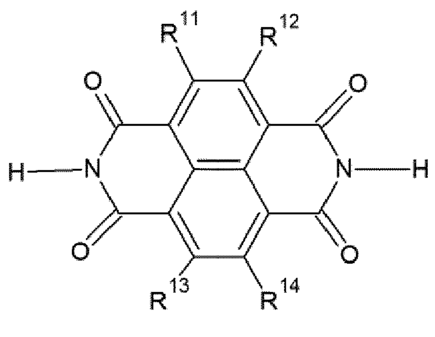

In Chemical Formula Ref, $R^{11}$, $R^{12}$, $R^{13}$, and $R^{14}$ are the same as in Chemical Formula 1.

As used herein, "energy disorder" may be measured by creating a morphology including 600 molecules through molecular dynamics simulation using OPLS4 as a force field parameter using Schrodinger's Desmond, sampling 200 molecules from this morphology, and then fitting the HOMO energy distribution obtained by DFT calculation using Schrodinger's Jaguar under the condition of b3lyp/LACV3P** to a normal distribution to calculate a width of the distribution

(σ = energy disorder).

In addition, the sublimation temperature of a compound may be measured by thermogravimetric analysis (TGA), and may be a temperature at which, for example, a weight loss of 10% of the compound compared to an initial weight of the compound occurs when thermogravimetric analysis is performed at a pressure of about 10 Pa or less (e.g., 0 Pa to about 10 Pa, about 0.01 Pa to about 10 Pa, about 0.1 Pa to about 10 Pa, about 1 Pa to about 10 Pa, etc.).

As used herein, reorganization energy and oscillator strength are values calculated at the DFT B3LYP/DGDZVP level using the Gaussian 09 program.

Hereinafter, a compound according to some example embodiments will be described. The compound is represented by Chemical Formula 1 and at least one of $X^1$ or $X^2$ of Chemical Formula 1 may be a substituent having a volume ranging from about 900 $bohr^3$ to about 3000 $bohr^3$ (V/molecule):

[Chemical Formula 1]

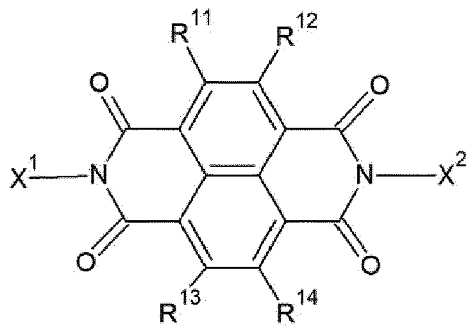

In Chemical Formula 1,

$X^1$ and $X^2$ may each independently be hydrogen, deuterium, tritium, a substituted or unsubstituted C1 to C10 alkyl group, a substituted or unsubstituted C1 to C10 alkoxy group, a substituted or unsubstituted C6 to C30 aryl group, or a substituted or unsubstituted C3 to C30 heteroaryl group,

at least one of $X^1$ or $X^2$ may be a substituent having a volume of about 900 $bohr^3$ to about 3000 $bohr^3$ (V/molecule), and the at least one of $X^1$ or $X^2$ may be a substituted or unsubstituted C6 to C30 aryl group or a substituted or unsubstituted C3 to C30 heteroaryl group, and

$R^{11}$, $R^{12}$, $R^{13}$, and $R^{14}$ may each independently be hydrogen, deuterium, tritium, a halogen, a cyano group, a C1 to C10 alkyl group, a C1 to C10 alkoxy group, or a C6 to C10 aryl group.

In some example embodiments, $X^1$ and $X^2$ may each independently be hydrogen, deuterium, tritium, a substituted or unsubstituted C1 to C10 alkyl group, a substituted or unsubstituted C1 to C10 alkoxy group, a substituted or unsubstituted C6 to C30 aryl group, or a substituted or unsubstituted C3 to C30 heteroaryl group, a C3 to C20 branched alkyl group, a C3 to C20 branched alkoxy group, a C3 to C20 cycloalkyl group, a C3 to C20 heterocycloalkyl group, a group including -Si($R^a$)($R^b$)($R^c$) (wherein $R^a$, $R^b$, and $R^c$ are each independently hydrogen, deuterium, tritium, a halogen, a cyano group, a linear or branched C1 to C10 alkyl group, or a C6 to C10 aryl group), a group including -Ge($R^a$)($R^b$)($R^c$) (wherein $R^a$, $R^b$, and $R^c$ are each independently hydrogen, deuterium, tritium, a halogen, a cyano group, a linear or branched C1 to C10 alkyl group, or a C6 to C10 aryl group), any of the substituents of Group 1 below, any of the substituents shown in the compounds of Group 2 below, or any combination thereof. In some example embodiments, at least one of $X^1$ or $X^2$ may be a substituent having a volume of about 900 $bohr^3$ to about 3000 $bohr^3$ (V/molecule). In some example embodiments, the at least one of $X^1$ or $X^2$ having the volume of about 900 $bohr^3$ to about 3000 $bohr^3$ (V/molecule) may be a substituted or unsubstituted C6 to C30 aryl group or a substituted or unsubstituted C3 to C30 heteroaryl group, although example embodiments are not limited thereto. In some example embodiments the at least one of $X^1$ or $X^2$ having the volume of about 900 $bohr^3$ to about 3000 $bohr^3$ (V/molecule) may be any of a substituted or unsubstituted C1 to C10 alkyl group, a substituted or unsubstituted C1 to C10 alkoxy group, a substituted or unsubstituted C6 to C30 aryl group, or a substituted or unsubstituted C3 to C30 heteroaryl group, a C3 to C20 branched alkyl group, a C3 to C20 branched alkoxy group, a C3 to C20 cycloalkyl group, a C3 to C20 heterocycloalkyl group, a group including -Si($R^a$)($R^b$)($R^c$) (wherein $R^a$, $R^b$, and $R^c$ are each independently hydrogen, deuterium, tritium, a halogen, a cyano group, a linear or branched C1 to C10 alkyl group, or a C6 to C10 aryl group), a group including -Ge($R^a$)($R^b$)($R^c$) (wherein $R^a$, $R^b$, and $R^c$ are each independently hydrogen, deuterium, tritium, a halogen, a cyano group, a linear or branched C1 to C10 alkyl group, or a C6 to C10 aryl group), any of the substituents of Group 1 below, any of the substituents shown in the compounds of Group 2 below, or any combination thereof.

In some example embodiments of the inventive concepts, based on a volume of a substituent of the compound represented by Chemical Formula 1 being within a certain range (e.g., based on the volume of at least one of $X^1$ or $X^2$ having a volume of about 900 $bohr^3$ to about 3000 $bohr^3$ (V/molecule), the photoelectric conversion efficiency of a device including the compound represented by Chemical Formula 1 may be greatly improved (e.g., thereby improving the photoelectric conversion performance of the device and/or improving the power consumption efficiency of the device without compromising photoelectric conversion performance thereof, thereby improving the functionality of the device) and the deposition stability of the compound and/or the device including same may be improved.

In Chemical Formula 1, at least one $X^1$ or $X^2$ may have a volume of greater than or equal to about 900 $bohr^3$ (V/molecule), greater than or equal to about 1000 $bohr^3$ (V/molecule), for example greater than or equal to about 1100 $bohr^3$ (V/molecule), greater than or equal to about 1200 $bohr^3$ (V/molecule), greater than or equal to about 1300 $bohr^3$ (V/molecule), greater than or equal to about 1400 $bohr^3$ (V/molecule), or greater than or equal to about 1500 $bohr^3$ (V/molecule), and may be less than or equal to about 2900 $bohr^3$ (V/molecule), for example less than or equal to about 2800 $bohr^3$ (V/molecule), less than or equal to about 2700 $bohr^3$ (V/molecule), less than or equal to about 2600 $bohr^3$ (V/molecule), less than or equal to about 2500 $bohr^3$ (V/molecule), or less than or equal to about 2400 $bohr^3$ (V/molecule).

In Chemical Formula 1, based on at least one of $X^1$ or $X^2$ including a substituent having the substituent volume within the above range, the photoelectric conversion efficiency of a device including the compound may be excellently improved, and the efficiency change of the compound and/or the device including the compound depending on the deposition rate is not significant, thereby ensuring process stability, thereby reducing the likelihood of process defects in the device and thus improving the reliability of the device.

In Chemical Formula 1, $X^1$ and $X^2$ may be the same or different from each other.

In Chemical Formula 1, $X^1$ and $X^2$ may be present symmetrically with the naphthalene structure of Chemical Formula 1 as the central axis.

The compound may have an energy disorder of less than or equal to about 0.22 eV, for example less than or equal to about 0.21 eV, or less than or equal to about 0.20 eV, and may be greater than or equal to about 0.01 eV, for example greater than or equal to about 0.02 eV, greater than or equal to about 0.03 eV, greater than or equal to about 0.04 eV, or greater than or equal to about 0.05 eV.

When the energy disorder is in the above range, it is easy to match the energy level of the compound, and thus the photoelectric conversion efficiency of the device can be excellently improved, and the efficiency change of the compound and/or the device including the compound depending on the deposition rate is not large, securing process stability, thereby reducing the likelihood of process defects in the device and thus improving the reliability of the device.

In Chemical Formula 1, at least one of $X^1$ or $X^2$ may include an electron donating group. The electron donating group may be a C3 to C20 branched alkyl group, a C3 to C20 branched alkoxy group, a C6 to C20 aryl group, a C3 to C20 cycloalkyl group, a C3 to C20 heteroaryl group, a C3 to C20 heterocycloalkyl group, or any combination thereof.

In Chemical Formula 1, at least one of $X^1$ or $X^2$ may include $-Si(R^a)(R^b)(R^c)$ (wherein $R^a$, $R^b$, and $R^c$ are each independently hydrogen, deuterium, tritium, a halogen, a cyano group, a linear or branched C1 to C10 alkyl group, or a C6 to C10 aryl group), $-Ge(R^a)(R^b)(R^c)$ (wherein $R^a$, $R^b$, and $R^c$ are each independently hydrogen, deuterium, tritium, a halogen, a cyano group, a linear or branched C1 to C10 alkyl group, or a C6 to C10 aryl group), or any combination thereof.

In Chemical Formula 1, about 10% to about 100% of the hydrogen atoms of at least one of $X^1$ or $X^2$ may be substituted with deuterium or tritium based on a total number of hydrogen atoms present in each substituent (e.g., in the at least one of $X^1$ or $X^2$). For example, about 10% or more, about 11% or more, about 12% or more, about 13% or more, about 14% or more, or about 15% or more and about 90% or less, about 89% or less, about 88% or less, about 87% or less, about 86% or less, or about 85% or less of the hydrogen atoms of at least one of $X^1$ or $X^2$ may be substituted with deuterium or tritium based on a total number of hydrogen atoms present in each substituent.

At least one of $X^1$ or $X^2$ may be a substituent selected from Group 1.

[Group 1]

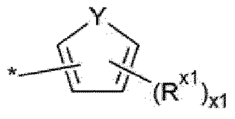
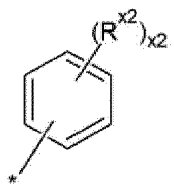
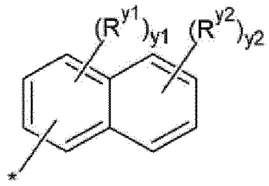
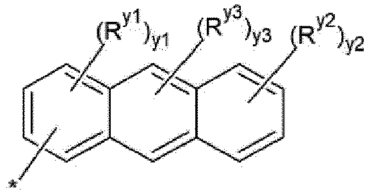

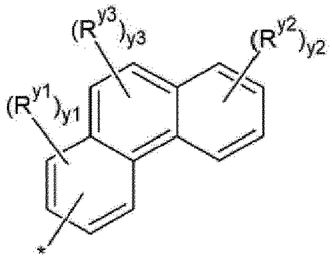
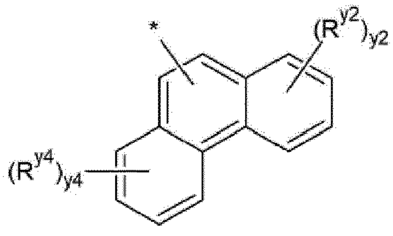
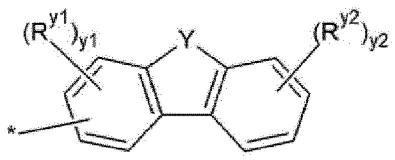

In Group 1,

Y is S, O, Se, Te, or CRR' (wherein R and R' are each independently hydrogen, deuterium, tritium, a C1 to C10 alkyl group, a C1 to C10 alkoxy group, or a C6 to C10 aryl group),
$R^{x1}$ is deuterium, tritium, a C3 to C20 branched alkyl group, a C3 to C20 branched alkoxy group, a C6 to C20 aryl group, a C3 to C20 cycloalkyl group, a C3 to C20 heteroaryl group, a C3 to C20 heterocycloalkyl group, -Si($R^a$)($R^b$)($R^c$) (wherein $R^a$, $R^b$, and $R^c$ are each independently hydrogen, deuterium, tritium, a halogen, a cyano group, a linear or branched C1 to C10 alkyl group, or a C6 to C10 aryl group), - Ge($R^a$)($R^b$)($R^c$) (wherein $R^a$, $R^b$, and $R^c$ are each independently hydrogen, deuterium, tritium, a halogen, a cyano group, a linear or branched C1 to C10 alkyl group, or a C6 to C10 aryl group), or any combination thereof,
x1 is an integer of 1 to 3,
$R^{x2}$ is deuterium, tritium, a C3 to C20 branched alkyl group, a C3 to C20 branched alkoxy group, a C6 to C20 aryl group, a C3 to C20 cycloalkyl group, a C3 to C20 heteroaryl group, a C3 to C20 heterocycloalkyl group, -Si($R^a$)($R^b$)($R^c$) (wherein $R^a$, $R^b$, and $R^c$ are each independently hydrogen, deuterium, tritium, a halogen, a cyano group, a linear or branched C1 to C10 alkyl group, or a C6 to C10 aryl group), - Ge($R^a$)($R^b$)($R^c$) (wherein $R^a$, $R^b$, and $R^c$ are each independently hydrogen, deuterium, tritium, a halogen, a cyano group, a linear or branched C1 to C10 alkyl group, or a C6 to C10 aryl group), or any combination thereof,
x2 is an integer of 1 to 5,
$R^{y1}$, $R^{y2}$, $R^{y3}$, and $R^{y4}$ are each independently hydrogen, deuterium, tritium, halogen, a cyano group, C1 to C10 linear alkyl group, C1 to C10 linear alkoxy group, C3 to C20 branched alkyl group, C3 to C20 branched alkoxy group, C6 to C20 aryl group, C3 to C20 cycloalkyl group, C3 to C20 heteroaryl group, C3 to C20 heterocycloalkyl group, -Si($R^a$)($R^b$)($R^c$) (wherein $R^a$, $R^b$, and $R^c$ are each independently hydrogen, deuterium, tritium, a halogen, a cyano group, a linear or branched C1 to C10 alkyl group, or a C6 to C10 aryl group), -Ge($R^a$)($R^b$)($R^c$) (wherein $R^a$, $R^b$, and $R^c$ are each independently hydrogen, deuterium, tritium, a halogen, a cyano group, a linear or branched C1 to C10 alkyl group, or a C6 to C10 aryl group), or any combination thereof,
y1 is an integer of 0 to 3,
y2 is an integer of 0 to 4,
y3 is an integer of 0 to 2,
y4 is an integer of 0 to 4, and
* is a linking point with Chemical Formula 1.

The compound represented by Chemical Formula 1 may have a reorganization energy of less than or equal to about 0.390 eV, for example less than or equal to about 0.385 eV and greater than or equal to about 0.05 eV, for example greater than or equal to about 0.06 eV, or greater than or equal to about 0.07 eV.
The compound represented by Chemical Formula 1 may have an oscillator strength of greater than or equal to about 0.40, for example, greater than or equal to about 0.42 and less than or equal to about 0.80.
The compound represented by Chemical Formula 1 may have a molecular weight (g/mol) of less than or equal

to about 1500, for example, less than or equal to about 1400, less than or equal to about 1300, less than or equal to about 1200, less than or equal to about 1100, or less than or equal to about 1000, and greater than or equal to about 500, for example greater than or equal to about 550, or greater than or equal to about 600. The deposition process can be easily performed within the above range.

The sublimation temperature (temperature for forming a film by vacuum deposition, also referred to as "deposition temperature") of the compound represented by Chemical Formula 1 may be less than or equal to about 340 °C, for example less than or equal to about 330 °C, less than or equal to about 320 °C, less than or equal to about 310 °C, less than or equal to about 300 °C, less than or equal to about 290 °C, less than or equal to about 280 °C, less than or equal to about 270 °C, less than or equal to about 260 °C, or less than or equal to about 255 °C, and may be for example, about 100 °C to about 270 °C or about 100 °C to about 255 °C. By having a sublimation temperature in the above range, there is little possibility of impurities being mixed in when forming a thin film by deposition. The sublimation temperature can be confirmed by thermogravimetric analysis (TGA), and may be a temperature at which, for example, a weight loss of 10% of the compound compared to an initial weight of the compound occurs when thermogravimetric analysis of the compound is performed at a pressure of 10 Pa or less.

In addition, a micro lens array (MLA) needs to be formed to concentrate light after manufacturing an organic photoelectric device during manufacture of an image sensor. This micro lens array requires a relatively high temperature (greater than or equal to about 160 °C, for example greater than or equal to about 170 °C, greater than or equal to about 180 °C, or greater than or equal to about 190 °C). The performance of the photoelectric devices (e.g., organic photoelectric devices) is required not to be deteriorated in these heat-treatment processes. The performance deterioration of the organic photoelectric device during the heat treatment of MLA may be caused not by chemical decomposition of an organic material but its morphology change. The morphology change is in general caused, when a material starts a thermal vibration due to a heat treatment, but a material having a firm molecule structure may not have the thermal vibration and be limited and/or prevented from the deterioration by the heat treatment. The compound represented by Chemical Formula 1 may be maintained stably due to improved thermal stability, even in the MLA heat treatment process by suppressing the vibration of molecules due to heat, thereby ensuring process stability and thereby reducing the likelihood of process defects in a layer, film, and/or device including the compound, and thereby improving the reliability of a device including the compound and/or a layer including the compound, a film including the compound, or the like.

The compound has transparency capable of transmitting light in the visible light region, and does not substantially absorb visible light in the wavelength range of from about 350 nm to about 750 nm, for example, about 400 nm or more and about 700 nm or less, about 450 nm or more and about 700 nm or less, or about 400 nm or more and less than about 500 nm. Herein, "not substantially absorbing" means that the absorption coefficient in the above wavelength range is less than or equal to about 40000 $cm^{-1}$. In some example embodiments, the compound may have an absorption coefficient of less than or equal to about 40000 $cm^{-1}$, for example less than or equal to about 20000 $cm^{-1}$ in a range of greater than or equal to about 400 nm and less than about 500 nm, for example about 420 to about 480 nm, for example at about 450 nm. Thereby, absorption in the blue light region may be significantly reduced and absorption selectivity in the green wavelength region may be improved, such that a photoelectric conversion device and/or photoelectric conversion layer including the compound may have improved absorption selectivity and thus photoelectric conversion selectivity in the green wavelength region, and thus a photoelectric conversion device and/or photoelectric conversion layer including the compound may have improved photoelectric conversion performance and/or efficiency with regard to light in the green wavelength region.

The compound may be used as an n-type semiconductor. The compound may have an energy bandgap of greater than or equal to about 2.8 eV, for example greater than or equal to about 3.0 eV, greater than or equal to about 3.1 eV, greater than or equal to about 3.2 eV, greater than or equal to about 3.3 eV, greater than or equal to about 3.4 eV, or greater than or equal to about 3.5 eV and may be less than or equal to about 4.0 eV, and may have a HOMO energy level may be greater than or equal to about 6.0 eV and less than about 8.5 eV.

Additionally, a difference between the HOMO energy level of the compound and the work function of the electrode (cathode) of the photoelectric device described later may be, for example, greater than or equal to about 1.5 eV, for example, greater than or equal to about 1.8 eV. Herein, the HOMO energy level refers to the absolute value of the HOMO energy level when the vacuum level is 0 eV. As a result, the compound can serve to block holes flowing in from the outside. Therefore, a hole blocking layer may not be disposed between the active layer and the cathode of the photoelectric device, thereby reducing complexity of the photoelectric device, thus improving miniaturization of the photoelectric device, reducing complexity and costs of a process of manufacturing the photoelectric device, and furthermore improving the reliability of the photoelectric device based on reducing the likelihood of process defects as a result of omitting, from the process of manufacturing the photoelectric device, a process of forming the hole blocking layer between the active layer and the cathode of the photoelectric device.

Examples of the compound represented by Chemical Formula 1 include compounds of Group 2.

[Group 2]

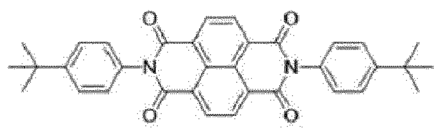

(1-1)

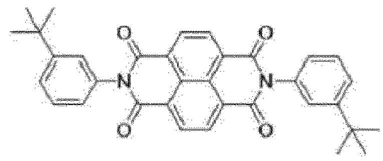

(1-2)

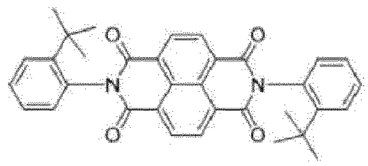

(1-3)

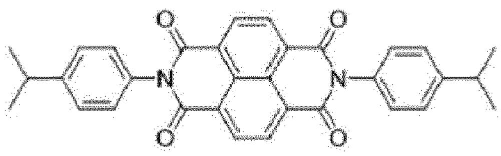

(1-4)

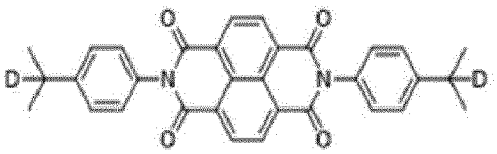

(1-5)

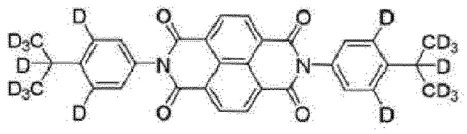

(1-6)

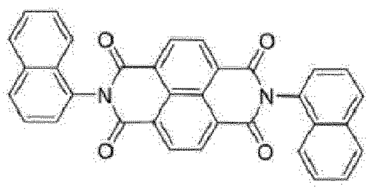

(1-7)

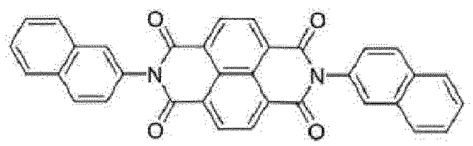

(1-8)

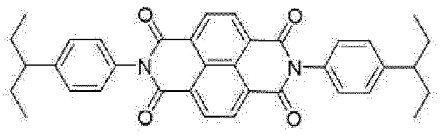

(1-9)

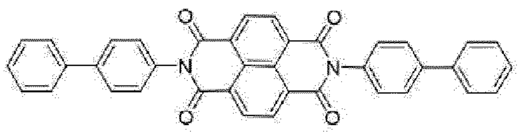

(1-10)

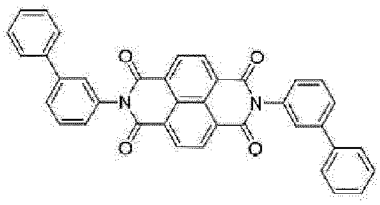

(1-11)

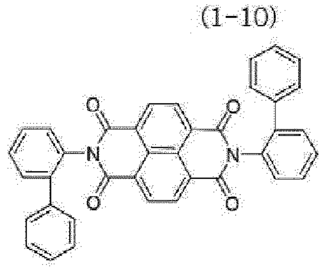

(1-12)

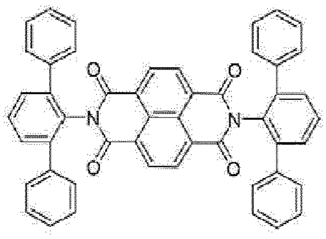

(1-13)

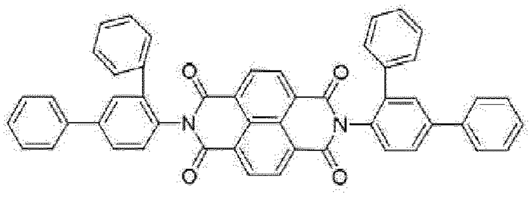

(1-14)

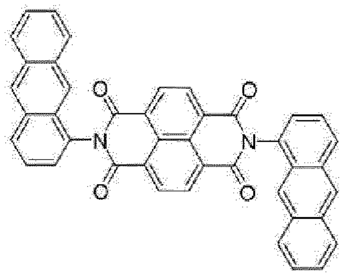

(1-15)

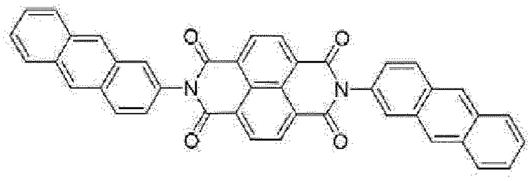

(1-16)

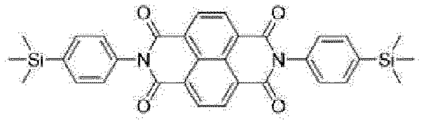

(1-17)

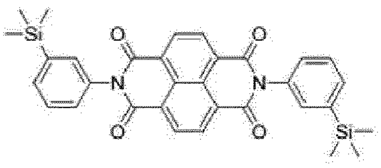

(1-18)

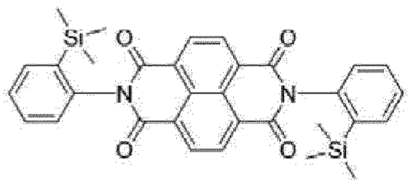

(1-19)

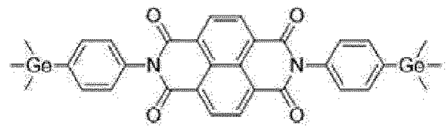

(1-20)

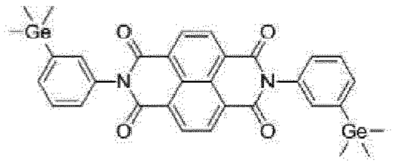

(1-21)

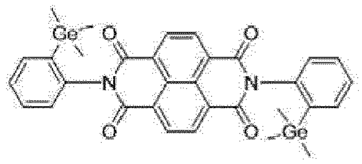

(1-22)

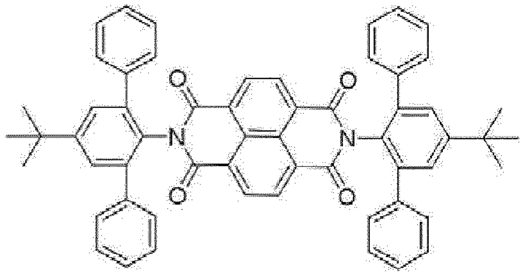

(1-23)

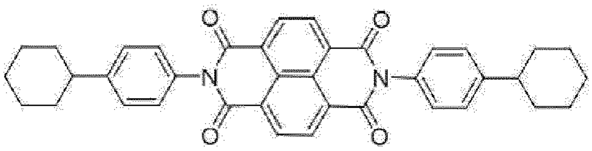

(1-24)

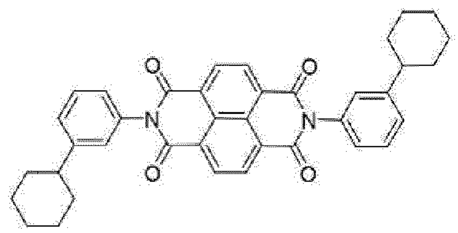

(1-25)

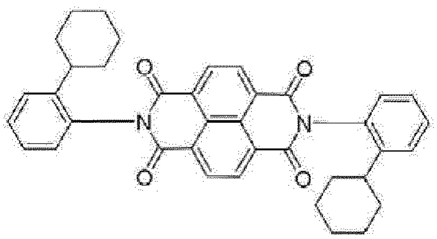

(1-26)

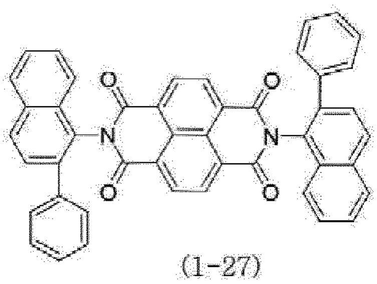

(1-27)

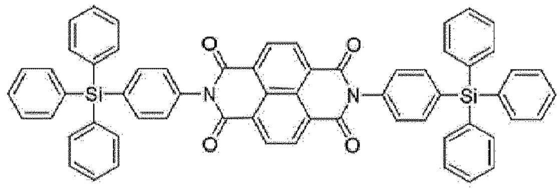

(1-28)

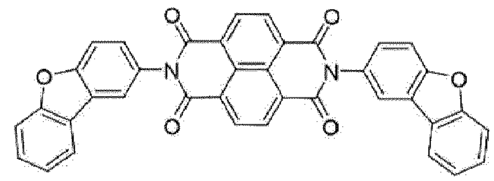

(1-29)

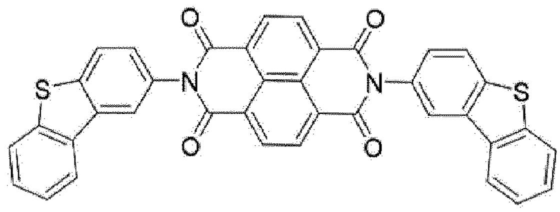

(1-30)

In some example embodiments, the compound represented by Chemical Formula 1 has a volume ratio of substituents $X^1$ and $X^2$ obtained according to Equation 1 and Equation 2, the volume ratio ranging from about 45% to about 85%:

[Equation 1]

$$\text{Volume } (V_b) \text{ of substituent} = V_a - V_{Ref}$$

[Equation 2]

$$\text{volume ratio of a substituent} = V_b / V_a$$

In Equations 1 and 2, $V_a$ is a volume of the compound, $V_b$ is a volume of the substituents $X^1$ and $X^2$, and $V_{Ref}$ is a volume of a compound represented by Chemical Formula Ref:

[Chemical Formula Ref]

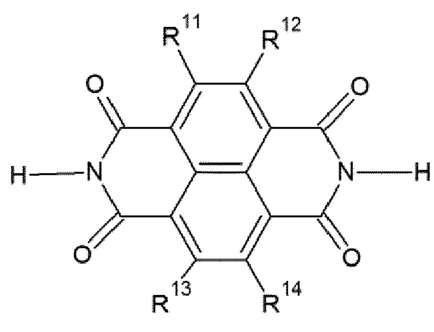

In Chemical Formula Ref, $R^{11}$, $R^{12}$, $R^{13}$, and $R^{14}$ are the same as in Chemical Formula 1.

Hereinafter, a photoelectric device according to some example embodiments including the aforementioned

compound will be described with reference to the drawings.

FIG. 1 is a cross-sectional view showing a photoelectric device according to some example embodiments.

Referring to FIG. 1, a photoelectric device 100 according to some example embodiments includes a first electrode 10 and a second electrode 20 (e.g., a first electrode 10 and a second electrode 20 facing each other), and an active layer 30 between the first electrode 10 and the second electrode 20.

One of the first electrode 10 or the second electrode 20 is an anode and the other is a cathode. At least one of the first electrode 10 or the second electrode 20 may be a light-transmitting electrode, and the light-transmitting electrode may be made of, for example, a transparent conductor such as indium tin oxide (ITO) or indium zinc oxide (IZO), or a metal thin layer of a thin single layer or multilayer. When one of the first electrode 10 or the second electrode 20 is a non-light-transmitting electrode, it may be made of, for example, an opaque conductor such as aluminum (Al).

The active layer 30 includes a p-type semiconductor and an n-type semiconductor to form a pn junction, and absorbs external light to generate excitons and then separates the generated excitons into holes and electrons.

The active layer 30 includes a p-type semiconductor compound represented by Chemical Formula 2 and an n-type semiconductor compound including the aforementioned compound represented by Chemical Formula 1.

[Chemical Formula 2]

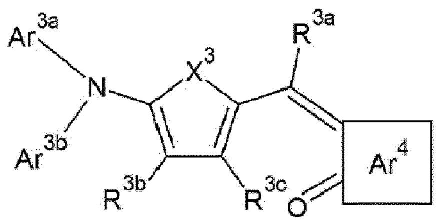

In Chemical Formula 2,

$X^3$ may be O, S, Se, Te, S(=O), $S(=O)_2$, $SiR^aR^b$, $GeR^cR^d$, or $CR^eR^f$ (wherein $R^a$, $R^b$, $R^c$, $R^d$, $R^e$, and $R^f$ are each independently hydrogen, deuterium, a substituted or unsubstituted C1 to C20 alkyl group, or a substituted or unsubstituted C6 to C20 aryl group, $R^a$, $R^b$, $R^c$, $R^d$, $R^e$ and $R^f$ are each independently present or a pair of $R^a$ and $R^b$, a pair of $R^c$ and $R^d$, or a pair of $R^e$ and $R^f$ is linked to form a spiro structure),

$Ar^{3a}$ and $Ar^{3b}$ may each independently be a substituted or unsubstituted C6 to C30 aryl group or a substituted or unsubstituted C3 to C30 heteroaryl group, wherein the aryl group or heteroaryl group is each independently present and is linked to each other to form a fused ring,

$Ar^4$ may be a substituted or unsubstituted C6 to C30 arene group, a substituted or unsubstituted C3 to C30 heteroarene group, or fused rings of two or more selected from these,

$R^{3a}$, $R^{3b}$, and $R^{3c}$ may each independently be hydrogen, deuterium, a substituted or unsubstituted C1 to C30 alkyl group, a substituted or unsubstituted C1 to C30 alkoxy group, a substituted or unsubstituted C6 to C30 aryl group, a substituted or unsubstituted C3 to C30 heteroaryl group, halogen, a cyano group, or any combination thereof, $R^{3b}$ and $R^{3c}$ may each independently be present or may be linked to each other to form a ring, and

$Ar^{3b}$ and $R^{3b}$ may optionally be linked to each other to form a fused ring.

The p-type semiconductor compound may include a compound represented by Chemical Formula 2A or Chemical Formula 2B.

[Chemical Formula 2A]

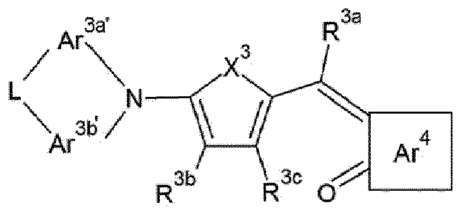

[Chemical Formula 2B]

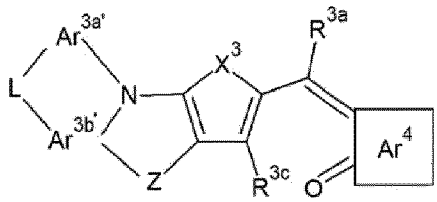

In Chemical Formula 2A and Chemical Formula 2B,

$X^3$ is O, S, Se, Te, S(=O), $S(=O)_2$, $SiR^aR^b$, $GeR^cR^d$, or $CR^eR^f$ (wherein $R^a$, $R^b$, $R^c$, $R^d$, $R^e$, and $R^f$ may each independently be hydrogen, deuterium, a substituted or unsubstituted C1 to C20 alkyl group, or a substituted or unsubstituted C6 to C20 aryl group, $R^a$, $R^b$, $R^c$, $R^d$, $R^e$, and $R^f$ are each independently present, a pair of $R^a$ and $R^b$, a pair of $R^c$ and $R^d$, or a pair of $R^e$ and $R^f$ is linked to each other to form a spiro structure),

$Ar^{3a'}$ and $Ar^{3b'}$ may each independently be a substituted or unsubstituted C6 to C30 arene group or a substituted or unsubstituted C3 to C30 heteroarene group,

$Ar^4$ may be a substituted or unsubstituted C6 to C30 arene group, a substituted or unsubstituted C3 to C30 heteroarene group, or fused rings of two or more selected from these,

L and Z may each independently be a single bond, O, S, Se, Te, SO, $SO_2$, $CR^fR^g$, $SiR^hR^i$, $GeR^jR^k$, $NR^l$, a substituted or unsubstituted C1 to C30 alkylene group, a substituted or unsubstituted C3 to C30 cycloalkylene group, a substituted or unsubstituted C6 to C30 arylene group, or any combination thereof (wherein $R^f$, $R^g$, $R^h$, $R^i$, $R^j$, $R^k$, and $R^l$ may each independently be hydrogen, deuterium, a substituted or unsubstituted C1 to C20 alkyl group, or a substituted or unsubstituted C6 to C20 aryl group, $R^f$, $R^g$, $R^h$, $R^i$, $R^j$, and $R^k$ are each independently present, or a pair of $R^f$ and $R^g$, a pair of $R^h$ and $R^i$, or a pair of $R^j$ and $R^k$ is linked to each other to form a spiro structure),

$R^{3a}$, $R^{3b}$, and $R^{3c}$ may each independently be hydrogen, deuterium, a substituted or unsubstituted C1 to C30 alkyl group, a substituted or unsubstituted C1 to C30 alkoxy group, a substituted or unsubstituted C6 to C30 aryl group, a substituted or unsubstituted C3 to C30 heteroaryl group, halogen, a cyano group, or any combination thereof, $R^{3b}$ and $R^{3c}$ may each independently be present or may be linked to each other to form a ring.

The active layer 30 may be configured to selectively absorb light in the red wavelength spectrum, green wavelength spectrum, or blue wavelength spectrum in the visible light region, or light in the infrared wavelength spectrum, where the red wavelength spectrum, the green wavelength spectrum, and the blue wavelength spectrum may each have a maximum absorption wavelength ($\lambda_{max}$) of greater than about 600 nm and less than about 750 nm, about 500 nm to about 600 nm, and greater than about 380 nm and less than 500 nm, and the infrared wavelength spectrum may exhibit a maximum absorption wavelength at greater than or equal to about 750 nm and less than or equal to about 3000 nm. Thus, an active layer 30 that is configured to selectively absorb light in the red wavelength spectrum, green wavelength spectrum, or blue wavelength spectrum in the visible light region, or light in the infrared wavelength spectrum may have a maximum absorption wavelength ($\lambda_{max}$) of greater than about 600 nm and less than about 750 nm, about 500 nm to about 600 nm, greater than about 380 nm and less than 500 nm, or greater than or equal to about 750 nm and less than or equal to about 3000 nm, respectively. For example, an active layer 30 that is configured to selectively absorb light in the green wavelength spectrum may have a maximum absorption wavelength in a wavelength range of greater than or equal to about 500 nm, for example greater than or equal to about 510 nm, greater than or equal to about 520 nm, greater than or equal to about 525 nm, greater than or equal to about 530 nm, or greater than or equal to about 535 nm, and less than or equal to about 590 nm, for example less than or equal to about 580 nm, less than or equal to about 570 nm, or less than or equal to about 560 nm.

The active layer 30 may exhibit a light absorption curve having a relatively narrow full width at half maximum (FWHM) of about 50 nm to about 200 nm, for example about 50 nm to about 150 nm, about 50 nm to about 120 nm, or about 50 nm to about 100 nm. Accordingly, the active layer 30 can have high selectivity for light in a specific wavelength region of the visible light region.

The active layer 30 may be a single layer or a multilayer. The active layer 30 may be, for example, an intrinsic layer (I layer), a p-type layer/I layer, an I layer/n-type layer, a p-type layer/I layer/n-type layer, a p-type layer/n-type layer, and the like.

The intrinsic layer (I layer) may include a p-type semiconductor compound and an n-type semiconductor compound in a volume ratio of about 1:100 to about 100:1. The p-type semiconductor compound and n-type semiconductor compound may be included in the active layer 30 in a volume ratio of about 1:50 to about 50:1, about 1:10 to about 10:1, about 1:5 to about 5:1, about 1:3 to about 3:1, about 1:2 to about 2:1, about 1:1.5 to about 1.5:1, about 1:1.2 to about 1.2:1, or about 1:1. When the p-type semiconductor compound and n-type semiconductor compound are included in the active layer 30 within the range, an exciton may be effectively produced, and a pn junction may be effectively formed.

The p-type layer may include the compound of Chemical Formula 2, and the n-type layer may include the compound of Chemical Formula 1.

The active layer 30 may have a thickness of about 1 nm to about 500 nm, for example about 20 nm to about 250 nm, about 30 nm to about 200 nm, about 50 nm to about 150 nm, about 70 nm to about 130 nm, about 80 nm to about 120 nm, or about 5 nm to about 300 nm. When the active layer 30 has a thickness within the range, the active layer 30 may effectively absorb light, effectively separate holes from electrons, and deliver them, thereby effectively improving photoelectric conversion efficiency. A desirable thickness of the active layer 30 may be, for example, determined by an absorption coefficient of the active layer 30, and may be, for example, a thickness being capable of absorbing light of at least about 70% or more, for example about 80% or more, and for another example about 90% or more.

In the photoelectric device 100, when light enters from the first electrode 10 and/or second electrode 20, and

when the active layer 30 absorbs light in a desired and/or in some example embodiments predetermined wavelength region, excitons may be produced from the inside. The excitons are separated into holes and electrons in the active layer 30, and the separated holes are transported to an anode that is one of the first electrode 10 or the second electrode 20 and the separated electrons are transported to the cathode that is the other of the first electrode 10 and the second electrode 20 so as to flow a current in (e.g., induce a current through at least a portion of) the photoelectric device 100.

Hereinafter, a photoelectric device according to some example embodiments is described with reference to FIG. 2.

FIG. 2 is a cross-sectional view showing a photoelectric device according to some example embodiments.

Referring to FIG. 2, a photoelectric device 200 according to some example embodiments includes a first electrode 10 and a second electrode 20 facing each other, and an active layer 30 between the first electrode 10 and the second electrode 20, like some example embodiments, including the example embodiments shown in FIG. 1.

However, the photoelectric device 200 according to some example embodiments, including the example embodiments shown in FIG. 2, further includes charge auxiliary layers 40 and 45 between the first electrode 10 and the active layer 30, and the second electrode 20 and the active layer 30, unlike some example embodiments, including the example embodiments shown in FIG. 1. The charge auxiliary layers 40 and 45 may facilitate the transfer of holes and electrons separated from the active layer 30, so as to increase efficiency.

The charge auxiliary layers 40 and 45 may be at least one selected from a hole injection layer (HIL) for facilitating hole injection, a hole transport layer (HTL) for facilitating hole transport, an electron blocking layer (EBL) for preventing electron transport, an electron injection layer (EIL) for facilitating electron injection, an electron transport layer (ETL) for facilitating electron transport, and a hole blocking layer (HBL) for preventing hole transport.

The charge auxiliary layers 40 and 45 may include, for example, an organic material, an inorganic material, or an organic/inorganic material. The organic material may be an organic compound having hole or electron characteristics, and the inorganic material may be, for example, a metal oxide such as molybdenum oxide, tungsten oxide, nickel oxide, and the like.

The hole injection layer (HIL) and/or hole transport layer (HTL) may include one selected from, for example, poly(3,4-ethylenedioxythiophene):poly(styrenesulfonate) (PEDOT:PSS), polyarylamine, poly(N-vinylcarbazole), polyaniline, polypyrrole, N,N,N',N'-tetrakis(4-methoxyphenyl)-benzidine (TPD), 4,4'-bis[N-(1-naphthyl)-N-phenyl-amino]biphenyl ($\alpha$-NPD), m-MTDATA, 4,4',4"-tris(N-carbazolyl)-triphenylamine (TCTA), and any combination thereof, but is not limited thereto.

The electron blocking layer (EBL) may include one selected from, for example, poly(3,4-ethylenedioxythiophene):poly(styrenesulfonate) (PEDOT:PSS), polyarylamine, poly(N-vinylcarbazole), polyaniline, polypyrrole, N,N,N',N'-tetrakis(4-methoxyphenyl)-benzidine (TPD), 4,4'-bis[N-(1 -naphthyl)-N-phenyl-amino]biphenyl ($\alpha$-NPD), m-MTDATA, 4,4',4"-tris(N-carbazolyl)-triphenylamine (TCTA), and any combination thereof, but is not limited thereto.

The electron injection layer (EIL) and/or electron transport layer (ETL) may include one selected from, for example, 1,4,5,8-naphthalene-tetracarboxylic dianhydride (NTCDA), bathocuproine (BCP), LiF, $Alq_3$, $Gaq_3$, $Inq_3$, $Znq_2$, $Zn(BTZ)_2$, $BeBq_2$, and any combination thereof, but is not limited thereto.

The hole blocking layer (HBL) may include one selected from, for example, 1,4,5,8-naphthalene-tetracarboxylic dianhydride (NTCDA), bathocuproine (BCP), LiF, $Alq_3$, $Gaq_3$, $Inq_3$, $Znq_2$, $Zn(BTZ)_2$, $BeBq_2$, and any combination thereof, but is not limited thereto.

Either one of the charge auxiliary layers 40 or 45 may be omitted.

The photoelectric device may be applied to various fields, for example a solar cell, an image sensor, a photodetector, a photo-sensor, and an organic light emitting diode (OLED), but is not limited thereto.

Hereinafter, an example of an image sensor including the organic photoelectric device is described referring to drawings. As an example of an image sensor, an organic CMOS image sensor is described.

FIG. 3 is a schematic top plan view showing an organic CMOS image sensor according to some example embodiments, and FIG. 4 is a cross-sectional view showing the organic CMOS image sensor of FIG. 3 according to some example embodiments.

Referring to FIGS. 3 and 4, an organic CMOS image sensor 300 according to some example embodiments, including the example embodiments shown in FIGS. 3 and 4 includes a semiconductor substrate 310 integrated with a photo-sensing device 50, a transmission transistor (not shown), a charge storage 55, a lower insulation layer 60, a color filter layer 70, an upper insulation layer 80 (also referred to herein as an insulation layer), and a photoelectric device 100. The photoelectric device 100 may be the photoelectric device according to any of the example embodiments and is on (e.g., directly or indirectly on) the semiconductor substrate 310.

The semiconductor substrate 310 may be a silicon substrate, and is integrated with a photo-sensing device 50, the transmission transistor (not shown), and the charge storage 55. The photo-sensing device 50 may include a blue photo-sensing device 50B and a red photo-sensing device 50R. The blue photo-sensing device 50B and the red photo-sensing device 50R may be photodiodes.

The photo-sensing device 50, the transmission transistor, and/or the charge storage 55 may be integrated in

each pixel, for example may be integrated in the semiconductor substrate 310 such that the photo-sensing device 50 (e.g., photo-sensing devices 50B and 50R) is located within a volume space defined by one or more outermost surfaces of the semiconductor substrate 310 and may be at least partially exposed by the semiconductor substrate 310 or may be enclosed within an interior of the semiconductor substrate 310, and as shown in the drawing, the photo-sensing device 50 may be respectively included in a blue pixel and a red pixel and the charge storage 55 may be included in a green pixel. The blue photo-sensing device 50B may be configured to sense (e.g., selectively sense, including selectively absorbing and photoelectrically converting) blue light which is light in a blue wavelength region, and the red photo-sensing device 50R may be configured to sense (e.g., selectively sense, including selectively absorbing and photoelectrically converting) red light which is light in a red wavelength region.

The blue photo-sensing device 50B may be configured to sense (e.g., selectively sense, including selectively absorbing and photoelectrically converting) blue light which is light in a blue wavelength region, and the red photo-sensing device 50R may be configured to sense (e.g., selectively sense, including selectively absorbing and photoelectrically converting) red light which is light in a red wavelength region.

The photo-sensing device 50 may be configured to sense (e.g., selectively sense) light, the information sensed by the photo-sensing device 50 may be transferred by the transmission transistor, the charge storage 55 is electrically connected to the photoelectric device 100, and the information of the charge storage 55 may be transferred by the transmission transistor.

In the drawings, the blue photo-sensing device 50B and the red photo-sensing device 50R are, for example, arranged in parallel without limitation, and the blue photo-sensing device 50B and the red photo-sensing device 50R may be stacked in a vertical direction.

A metal wire (not shown) and a pad (not shown) are formed on the semiconductor substrate 310. In order to decrease signal delay, the metal wire and pad may be made of a metal having low resistivity, for example, aluminum (Al), copper (Cu), silver (Ag), and alloys thereof, but are not limited thereto. Further, it is not limited to the structure, and the metal wire and pad may be positioned under the photo-sensing device 50.

The lower insulation layer 60 is formed on the metal wire and the pad. The lower insulation layer 60 may be made of an inorganic insulating material such as a silicon oxide and/or a silicon nitride, or a low dielectric constant (low K) material such as SiC, SiCOH, SiCO, and SiOF. The lower insulation layer 60 has a trench exposing the charge storage 55. The trench may be filled with fillers.

A color filter layer 70 is formed on the lower insulation layer 60. In some example embodiments, the color filter layer 70 may be located between the photoelectric device 100 and the semiconductor substrate 310. The color filter layer 70 includes a blue filter 70B formed in the blue pixel and configured to selectively transmit blue light and a red filter 70R formed in the red pixel and configured to selectively transmit red light. In some example embodiments, a cyan filter and a yellow filter may be disposed instead of the blue filter 70B and red filter 70R. In some example embodiments, including the example embodiments shown in FIGS. 3 and 4, a green filter is not included, but a green filter may be further included.

The color filter layer 70 may be omitted. For example, when the blue photo-sensing device 50B and the red photo-sensing device 50R are stacked in a vertical direction, the blue photo-sensing device 50B and the red photo-sensing device 50R may selectively absorb and/or sense light in each wavelength region depending on their stack depth, and the color filter layer 70 may not be equipped.

The upper insulation layer 80 is formed on the color filter layer 70. The upper insulation layer 80 eliminates a step caused by the color filter layer 70 and smoothens the surface. The upper insulation layer 80 and the lower insulation layer 60 may include a contact hole (not shown) exposing a pad, and a through-hole 85 exposing the charge storage 55 of the green pixel.

The aforementioned photoelectric device 100 is formed on (e.g., directly or indirectly on) the upper insulation layer 80. The photoelectric device 100 includes the first electrode 10, the active layer 30, and the second electrode 20 as described above.

The first electrode 10 and the second electrode 20 may be transparent electrodes, and the active layer 30 is the same as described above. The active layer 30 may selectively absorb and/or sense light in a green wavelength region and replaces a color filter of a green pixel. Accordingly, the photoelectric device 100 may be on the semiconductor substrate 310 and may be configured to selectively sense light in the green wavelength region.

When light enters from the second electrode 20, the light in a green wavelength region may be mainly absorbed in the active layer 30 and photoelectrically converted, while the light in the rest of the wavelength regions passes through first electrode 10 and may be sensed in the photo-sensing device 50.

As described above, the photoelectric devices configured to selectively absorb light in a green wavelength region are stacked and thereby a size of an image sensor may be decreased and a down-sized image sensor may be realized.

As described above, the compound represented by the Chemical Formula 1 may be used as a n-type semiconductor compound, aggregation between compounds in a thin film state is inhibited, and thereby light absorption

characteristics depending on a wavelength may be maintained. Thereby, green wavelength selectivity may be maintained, crosstalk caused by unnecessary absorption of other light except a green wavelength region may be decreased and sensitivity may be increased.

In some example embodiments, in FIG. 4, additional color filters may be further disposed on the photoelectric device 100. The additional color filters may include a blue filter 70B and a red filter 70R or a cyan filter and a yellow filter.

The organic CMOS image sensor with the color filters disposed on the photoelectric device is shown in FIG. 5.

FIG. 5 is a schematic cross-sectional view showing an organic CMOS image sensor according to some example embodiments. Referring to FIG. 5, an organic CMOS image sensor 400 has the same structure as FIG. 4 except that a color filter layer 72 including the blue filter 72B and the red filter 72R is disposed on the photoelectric device 100 such that the photoelectric device 100 is between the color filter layer 72 and the semiconductor substrate 310. Instead of the blue filter 72B and the red filter 72R, the cyan filter and the yellow filter may be disposed respectively.

In FIGS. 4 and 5, the photoelectric device 100 of FIG. 1 is included, but it is not limited thereto, and thus the photoelectric device 200 of FIG. 2 may be applied in the same manner.

FIG. 6 is a cross-sectional view showing an organic CMOS image sensor 500 to which the photoelectric device 200 is applied according to some example embodiments.

Referring to FIG. 6, the organic CMOS image sensor 500 includes a semiconductor substrate 310 integrated with photo-sensing devices 50B and 50R, a transmission transistor (not shown), a charge storage 55, a lower insulation layer 60, an upper insulation layer 80, and a photoelectric device 200, like some example embodiments, including the example embodiments shown in FIG. 4. However, the organic CMOS image sensor 500 according to some example embodiments, including the example embodiments shown in FIG. 6, includes the photoelectric device 200, unlike some example embodiments, including the example embodiments shown in FIG. 4, which include the photoelectric device 100.

FIG. 7 is a schematic view showing an organic CMOS image sensor according to some example embodiments.

Referring to FIG. 7, the organic CMOS image sensor 600 includes a semiconductor substrate 310 integrated with photo-sensing devices 50B and 50R, a transmission transistor (not shown), and a charge storage 55, an insulation layer 80, and a photoelectric device 100, like some example embodiments, including the example embodiments illustrated in FIG. 4.

However, the organic CMOS image sensor 600 according to some example embodiments includes the blue photo-sensing device 50B and the red photo-sensing device 50R that are stacked in a vertical direction (e.g., perpendicular to a direction in which the upper surface of the semiconductor substrate 310 extends as shown in FIG. 7) in the semiconductor substrate 310 and does not include a color filter layer 70, unlike some example embodiments, including the example embodiments shown in FIG. 4. For example, each red photo-sensing device 50R of a plurality of red photo-sensing devices 50R integrated in the semiconductor substrate 310 may be stacked in a vertical direction with a separate blue photo-sensing device 50B of a plurality of blue photo-sensing devices 50B in the semiconductor substrate 310. The blue photo-sensing device 50B and the red photo-sensing device 50R are electrically connected with the charge storage 55, and the information of the charge storage 55 may be transferred by the transmission transistor (not shown). The blue photo-sensing device 50B and the red photo-sensing device 50R may selectively absorb light in each wavelength region depending on a stack depth.

As described above, the photoelectric devices selectively absorbing light in a green wavelength region are stacked and the red photo-sensing device and the blue photo-sensing device are stacked, and thereby a size of an image sensor may be decreased and a down-sized image sensor may be realized. As described above, the photoelectric device 100 has improved green wavelength selectivity, and crosstalk caused by unnecessary absorption of light in a wavelength region except green may be decreased while increasing sensitivity.

In FIG. 7, the photoelectric device 100 of FIG. 1 is included, but it is not limited thereto, and thus the photoelectric device 200 of FIG. 2 may be applied in the same manner.

FIG. 8 is a schematic view showing an organic CMOS image sensor according to some example embodiments and FIG. 9 is a cross-sectional view of the organic CMOS image sensor of FIG. 8 according to some example embodiments.

Referring to FIGS. 8 and 9, the organic CMOS image sensor 700 according to some example embodiments includes a green photoelectric device G configured to selectively absorb light in a green wavelength region, a blue photoelectric device B configured to selectively absorb light in a blue wavelength region, and a red photoelectric device R configured to selectively absorb light in a red wavelength region that are stacked. For example, the organic CMOS image sensor 700 may include a green photoelectric device configured to selectively sense light in a green wavelength region, a blue photoelectric device configured to selectively sense light in a blue wavelength region, and a red photoelectric device configured to selectively sense light in a red wavelength region, where the green photoelectric device, the blue photoelectric device, and the red photoelectric device are stacked as shown in at least FIG. 8. As shown, the photoelectric devices 100a to 100c may be stacked in a vertical direction on the semiconductor substrate 310, such that the photoelectric devices 100a to 100c at least partially overlap each other in a vertical direction that is perpendicular to an upper surface 40s of the semiconductor substrate 310, but example embodiments are not limited thereto.

The organic CMOS image sensor 700 according to some example embodiments includes a semiconductor substrate 310, a lower insulation layer 60, an intermediate insulation layer 65, an upper insulation layer 80, a first device (i.e., photoelectric device, the same below) 100a, a second device 100b, and a third device 100c.

The semiconductor substrate 310 may be a silicon substrate, and a transmission transistor (not shown) and charge storages 155a, 155b, and 155c are integrated therein.

Metal wires (not shown) and pads (not shown) are formed on the semiconductor substrate 310, and the lower insulation layer 60 is formed on the metal wires and the pads.

The first device 100a, the second device 100b, and the third device 100c are sequentially formed on the lower insulation layer 60.

Any one of the first, second, and third devices 100a, 100b, and 100c may be the photoelectric devices 100 or 200 (green photoelectric device according to example embodiments) of FIG. 1 or 2, and the other two of the first, second, and third devices 100a, 100b, and 100c (a red photoelectric device and a blue photoelectric device) may have the same structure as the photoelectric devices 100 and 200, but an active layer 30 therein selectively absorbs light in a red or blue wavelength region to photoelectrically convert the light. Detailed descriptions of the photoelectric devices 100 and 200 are the same as described above. The first electrode 10 and the second electrode 20 of the photoelectric devices 100 and 200, the red photoelectric device and the blue photoelectric device may be connected to the charge storages 155a, 155b, and 155c.

The active layer 30 of the first device 100a may selectively absorb light in any one wavelength region of red, blue, or green to photoelectrically convert the light. For example, the first device 100a may be a red photoelectric conversion device configured to selectively sense light in a red wavelength region. The first electrode 10 or the second electrode 20 of the first device 100a may be electrically connected to the first charge storage 155a. A "photoelectric conversion device" may be interchangeably referred to herein as a "photoelectric device."

The intermediate insulation layer 65 may be formed on the first device 100a and the second device 100b may be formed on the intermediate insulation layer 65.

The active layer 30 of the second device 100b may selectively absorb light in any one wavelength region of red, blue, or green to photoelectrically convert the light. For example, the second device 100b may be a green photoelectric conversion device configured to selectively sense light in a green wavelength region. In another example, the second device 100b may be a blue photoelectric conversion device configured to selectively sense light in a blue wavelength region. The first electrode 10 or the second electrode 20 of the second device 100b may be electrically connected to the second charge storage 155b.

The upper insulation layer 80 is formed on the second device 100b. The lower insulation layer 60, the intermediate insulation layer 65, and the upper insulation layer 80 have a plurality of through-holes 85a, 85b, and 85c exposing the charge storages 155a, 155b, and 155c.

The third device 100c is formed on the upper insulation layer 80. The active layer 30 of the third device 100c may selectively absorb light in any one wavelength region of red, blue, or green to photoelectrically convert the light. For example, the third device 100c may be a blue photoelectric conversion device configured to selectively sense light in a blue wavelength region. In another example, the third device 100c may be a green photoelectric conversion device configured to selectively sense light in a green wavelength region. The first electrode 10 or the second electrode 20 of the third device 100c may be electrically connected to the third charge storage 155c.

A focusing lens (not shown) may be further formed on the third device 100c. The focusing lens may control direction of incident light and gather the light in one region. The focusing lens may have a shape of, for example, a cylinder or a hemisphere, but is not limited thereto.

In the drawing, a structure in which the first device 100a, the second device 100b, and the third device 100c are sequentially stacked, but is not limited thereto, and the stacking order may be variously changed.

As described above, the first device 100a, the second device 100b, and the third device 100c that absorb light in different wavelength regions have a stacked structure, further reducing a size of the image sensor, implementing a down-sized image sensor, and simultaneously increasing sensitivity and reducing a crosstalk.

FIG. 10 is a perspective view schematically showing an example of an organic CMOS image sensor according to some example embodiments, and FIG. 11 schematically shows a view of a cross-section of an example of the organic CMOS image sensor of FIG. 10 taken along line XI-XI' of FIG. 10 according to some example embodiments.

Referring to FIGS. 10 and 11, the organic CMOS image sensor 800 according to some example embodiments, as in some example embodiments, including the example embodiments shown in at least FIG. 4, includes the semiconductor substrate 310 and the insulation layer 80. The organic CMOS image sensor 800 may include an optical filter 250 which may be configured to selectively transmit light of various wavelength regions (e.g., selectively transmit visible light). In some example embodiments, the optical filter 250 may be omitted. The organic CMOS image sensor 800 includes multiple photoelectric devices 100aa, 100bb, 100cc, and 100dd which are stacked horizontally on the semiconductor substrate 310, for example stacked in a direction that is parallel to the upper surface 40s of the semiconductor substrate 310 such that the photoelectric devices 100aa to 100dd at least partially overlap each other in a horizontal

direction that is parallel to the upper surface 40s of the semiconductor substrate 310.

As shown, for example, in FIG. 10, photoelectric devices 100aa and 100cc may be blue photoelectric devices configured to selectively sense light in a blue wavelength region, photoelectric device 100bb may be a green photoelectric device configured to selectively sense light in a green wavelength region, and photoelectric device 100dd may be a red photoelectric device configured to selectively sensor light in a red wavelength region, but example embodiments are not limited thereto, and each of the photoelectric devices 100aa to 100dd may be configured to selectively sense light of any wavelength region, including visible or non-visible (e.g., infrared or ultraviolet) light, provided that at least one of the photoelectric devices 100aa to 100dd is the green photoelectric device according to some example embodiments, such as the photoelectric device 100 or 200 as shown in FIG. 1 or 2.

As shown, the photoelectric devices 100aa to 100dd may be aligned side by side along a plane direction (e.g., XY direction) of the semiconductor substrate 310 and respectively connected, via respective through-holes 85, to respective charge storages 155a to 155d integrated in the semiconductor substrate 310. As shown, the respective first electrodes 210a to 210d, active layers 230a to 230d, and second electrodes 220a to 220d of the photoelectric devices 100aa to 100dd may be aligned side by side along a plane direction (e.g., XY direction) of the semiconductor substrate 310 (e.g., overlap in the horizontal direction).

At least one of the photoelectric devices 100aa to 100dd may each independently be the photoelectric devices 100 or 200 (green photoelectric device according to example embodiments) of FIG. 1 or 2, and the others (including a red photoelectric device and a blue photoelectric device) may have the same structure as the photoelectric devices 100 and 200, but an active layer 30 therein selectively absorbs light in a red or blue wavelength region to photoelectrically convert the light. Detailed descriptions of the photoelectric devices 100 and 200 are the same as described above. The first electrode 10 and the second electrode 20 of the photoelectric devices 100 and 200, the red photoelectric device, and the blue photoelectric device may be connected to the charge storages 155a, 155b, 155c, and 155d.

The photoelectric device 100aa includes a first electrode 210a, an active layer 230a, and a second electrode 220a. The photoelectric device 100bb includes a first electrode 210b, an active layer 230b, and a second electrode 220b. The photoelectric device 100cc includes a first electrode 210c, an active layer 230c, and a second electrode 220c. The photoelectric device 100dd includes a first electrode 210d, an active layer 230d, and a second electrode 220d.

The first electrodes 210a to 210d may each be the same as the first electrodes 10 of the photoelectric devices 100 and 200 of FIG. 1 or 2. The second electrodes 220a to 220d may each be the same as the second electrodes 20 of the photoelectric devices 100 and 200 of FIG. 1 or 2. The active layers 230a to 230d may each be the same as the active layers 30 of the photoelectric devices 100 and 200 of FIG. 1 or 2. At least some of the active layers 230a to 230d may have different material compositions and may be configured to sense (e.g., selectively absorb and photoelectrically convert) light in different wavelength regions. The active layers 230a and 230c may each be configured to selectively absorb light in the blue wavelength region and photoelectrically convert the absorbed light, the active layer 230b may be configured to selectively absorb light in the green wavelength region and photoelectrically convert the absorbed light, and the active layer 230d may be configured to absorb light in the red wavelength region and photoelectrically convert the absorbed light.

In some example embodiments, some or all of the second electrodes 220a to 220d may be separate portions of a single, continuous piece of material that extends between each of the photoelectric devices 100aa to 100dd to establish a common electrode, while the first electrodes 210a to 210d may be separate pieces of material in separate photoelectric devices 100aa to 100dd. In some example embodiments, some or all of the first electrodes 210a to 210d may be separate portions of a single, continuous piece of material that extends between each of the photoelectric devices 100aa to 100dd to establish a common electrode, while the second electrodes 220a to 220d may be separate pieces of material in separate photoelectric devices 100aa to 100dd.

The image sensor absorbs light in an appropriate wavelength region and may show both improved sensitivity (YSNR10) and color reproducibility ($\Delta E^*ab$) despite a stacked structure.

Herein, the YSNR10 indicates sensitivity of the image sensor, which is measured in a method described in Juha Alakarhu's "Image Sensors and Image Quality in Mobile Phones" printed in 2007 International Image Sensor Workshop (Ogunquit Maine, USA) but minimum illuminance expressed by lux at a ratio of 10 between signal and noise. Accordingly, the smaller the YSNR10 is, the higher sensitivity is.

On the other hand, the color reproducibility ($\Delta E^*ab$) shows a difference from standard colors in an X-Rite chart, and the $\Delta E^*ab$ (also indicated as simply $\Delta E$) is defined as a distance between two points on a $L^*a^*b^*$ color space by CIE (Commission International de L' Eclairage) in 1976. For example, the color difference may be calculated according to Equation 3.

[Equation 3]

$$\Delta E = \sqrt{(\Delta L^*)^2 + (\Delta a^*)^2 + (\Delta b^*)^2}$$

In Equation 3,

$\Delta E$ denotes the color reproducibility (also denoted as $\Delta E^*ab$ herein),
$\Delta L^*$ denotes a change of a color coordinate L* compared with the color coordinate L* at room temperature (about 20 °C to about 25 °C),
$\Delta a^*$ denotes a change of a color coordinate a* compared with the color coordinate a* at room temperature (about 20 °C to about 25 °C), and
$\Delta b^*$ denotes a change of a color coordinate b* compared with the color coordinate b* at room temperature (about 20 °C to about 25 °C).

In order to manufacture an image sensor having high sensitivity and high color reproducibility, YSNR10≤100lux at $\Delta E^*ab \leq 3$, and herein, the compound may realize YSNR10≤100 lux of sensitivity at color reproducibility of $\Delta E^*ab \leq 3$.

The image sensor may be applied to (e.g., included in) various electronic devices, for example, a mobile phone, a digital camera, and the like but is not limited thereto.

FIG. 12 is a block diagram of a digital camera including an image sensor according to some example embodiments.

Referring to FIG. 12, a digital camera 1000 includes a lens 1010, an image sensor 1020, a motor 1030, and an engine 1040. The image sensor 1020 may be one of image sensors according to some example embodiments, including any of the example embodiments shown in FIGS. 3 to 11.

The lens 1010 concentrates incident light on the image sensor 1020. The image sensor 1020 generates RGB data for received light through the lens 1010.

In some example embodiments, the image sensor 1020 may interface with the engine 1040.

The motor 1030 may adjust the focus of the lens 1010 or perform shuttering in response to a control signal received from the engine 1040. The engine 1040 may control the image sensor 1020 and the motor 1030.

The engine 1040 may be connected to a host/application 1050.

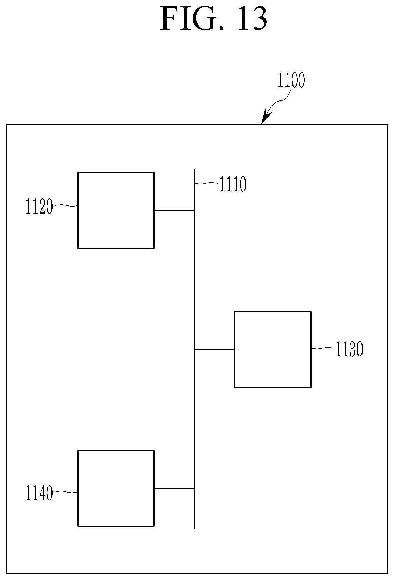

FIG. 13 is a schematic view showing an electronic device according to some example embodiments.

Referring to FIG. 13, an electronic device 1100 may include a processor 1120, a memory 1130, and an image sensor 1140 that are electrically coupled together via a bus 1110.

The image sensor 1140 may be an image sensor, organic CMOS image sensor, or the like according to any of the example embodiments, including any of the example embodiments shown in FIGS. 3 to 11 of the present application. The memory 1130 may be a non-transitory computer readable medium and may store a program of instructions. The memory 1130 may be a nonvolatile memory, such as a flash memory, a phase-change random access memory (PRAM), a magneto-resistive RAM (MRAM), a resistive RAM (ReRAM), or a ferro-electric RAM (FRAM), or a volatile memory, such as a static RAM (SRAM), a dynamic RAM (DRAM), or a synchronous DRAM (SDRAM). The processor 1120 may execute the stored program of instructions to perform one or more functions. For example, the processor 1120 may be configured to process electrical signals generated by the image sensor 1140. The processor 1120 may include processing circuitry such as hardware including logic circuits; a hardware/software combination such as a processor executing software; or any combination thereof. For example, the processing circuitry more specifically may include, but is not limited to, a central processing unit (CPU), an arithmetic logic unit (ALU), a digital signal processor, a microcomputer, a field programmable gate array (FPGA), a System-on-Chip (SoC), a programmable logic unit, a microprocessor, application-specific integrated circuit (ASIC), etc. The processor 1120 may be configured to generate an output (e.g., an image to be displayed on a display interface) based on such processing.

One or more of the processor 1120, memory 1130, motor 1030, engine 1040, or host/application 1050 may be included in, include, and/or implement one or more instances of processing circuitry such as hardware including logic circuits, a hardware/software combination such as a processor executing software; or any combination thereof. In some example embodiments, said one or more instances of processing circuitry may include, but are not limited to, a central processing unit (CPU), an application processor (AP), an arithmetic logic unit (ALU), a graphic processing unit (GPU), a digital signal processor, a microcomputer, a field programmable gate array (FPGA), a System-on-Chip (SoC), a programmable logic unit, a microprocessor, or an application-specific integrated circuit (ASIC), etc. In some example embodiments, any of the memories, memory units, or the like as described herein may include a non-transitory computer readable storage device, for example a solid state drive (SSD), storing a program of instructions, and the one or more

instances of processing circuitry may be configured to execute the program of instructions to implement the functionality of some or all of any of the processor 1120, memory 1130, motor 1030, engine 1040, or host/application 1050, or the like according to any of the example embodiments as described herein.

Hereinafter, some example embodiments are illustrated in more detail with reference to examples. However, these examples are non-limiting, and the inventive concepts are not limited thereto.

## Synthesis Examples

### Synthesis Example 1-1: Synthesis of Compound Represented by Chemical Formula 1-1

[Chemical Formula 1-1]

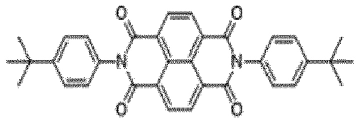

[Reaction Scheme 1-1]

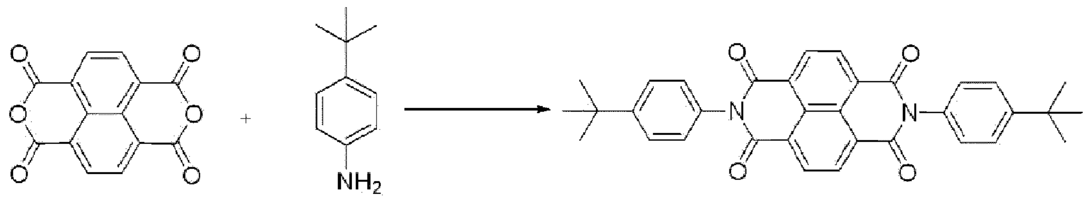

A mixture of naphthalene tetracarboxylic dianhydride (1 eq.) and 4-(tert-butyl)aniline (2.2 eq.) is dissolved in dimethylformamide (DMF) solvent to obtain a solution. The solution is placed in a two-necked round-bottomed flask and stirred at 180 °C for 24 hours. Then, the temperature is lowered to room temperature (24 °C), methanol is added thereto, the product is precipitated, and the product is filtered to obtain a powder-like material. The material is then washed with methanol several times and purified by recrystallization using ethyl acetate and dimethyl sulfoxide (DMSO). The obtained product is then placed in an oven and dried in vacuum at a temperature of 80 °C for 24 hours to obtain a compound represented by Chemical Formula 1-1. A yield is 50% or more.

The obtained compound is confirmed through mass and HPLC analysis.

HRMS(MALDI) calcd for $C_{34}H_{30}N_2O_4$: m/z: 530.2206, Found: 530.2204

### Synthesis Example 1-2: Synthesis of Compound Represented by Chemical Formula 1-2

[Chemical Formula 1-2]

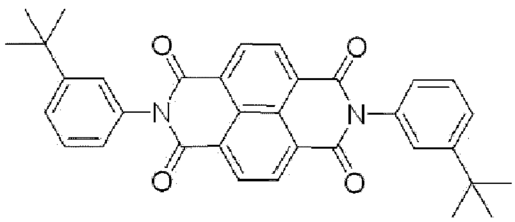

[Reaction Scheme 1-2]

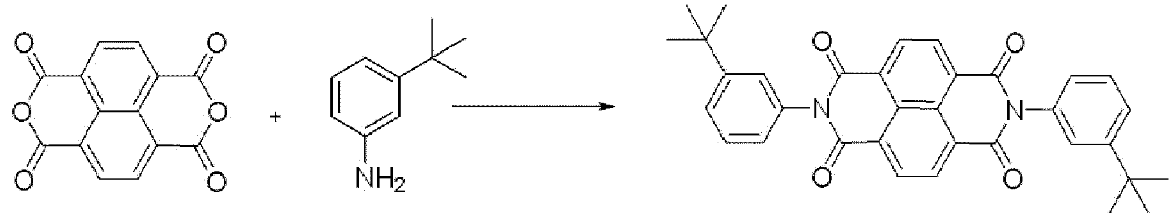

A mixture of naphthalene tetracarboxylic dianhydride (1 eq.) and 3-(tert-butyl)aniline (2.2 eq.) is dissolved in dimethylformamide (DMF) solvent to obtain a solution. The solution is placed in a two-necked round-bottomed flask and stirred at 180 °C for 24 hours. Then, the temperature is lowered to room temperature, methanol is added thereto, the product is precipitated, and the product is filtered to obtain a powder-like material. The material is then washed with methanol several times and purified by recrystallization using ethyl acetate and dimethyl sulfoxide (DMSO). The obtained product is then placed in an oven and dried in vacuum at a temperature of 80 °C for 24 hours to obtain a compound represented by Chemical Formula 1-2. A yield is 38%.

The obtained compound is confirmed through mass and HPLC analysis.

HRMS(MALDI) calcd for C34H30N2O4: m/z: 530.2206, Found: 530.2207

**Synthesis Example 1-3: Synthesis of Compound Represented by Chemical Formula 1-3 (Compound 1-4)**

[Chemical Formula 1-3]

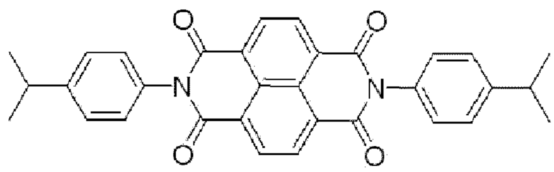

[Reaction Scheme 1-3]

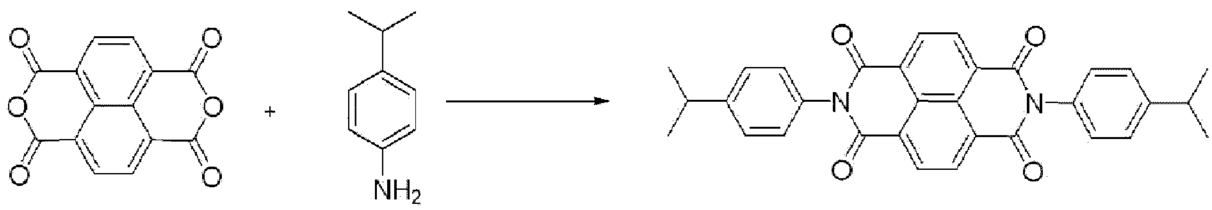

A mixture of naphthalene tetracarboxylic dianhydride (1 eq.) and 4-isopropylaniline (2.2 eq.) is dissolved in dimethylformamide (DMF) solvent to obtain a solution. The solution is placed in a two-necked round-bottomed flask and stirred at 180 °C for 24 hours. Then, the temperature is lowered to room temperature, methanol is added thereto, the product is precipitated, and the product is filtered to obtain a powder-like material. The material is then washed with methanol several times and purified by recrystallization using ethyl acetate and dimethyl sulfoxide (DMSO). The obtained product is then placed in an oven and dried in vacuum at a temperature of 80 °C for 24 hours to obtain a compound represented by Chemical Formula 1-3 (Compound 1-4 of Group 2). A yield is 50% or more.

The obtained compound is confirmed through mass and HPLC analysis. HRMS(MALDI) calcd for C32H26N2O4: m/z: 502.1893, Found: 502.1892

**Synthesis Example 1-4: Synthesis of Compound Represented by Chemical Formula 1-4 (Compound 1-17)**

[Chemical Formula 1-4]

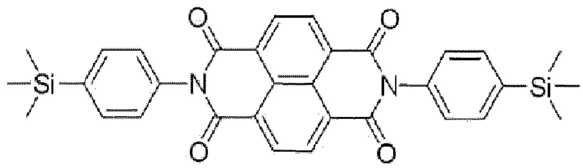

[Reaction Scheme 1-4]

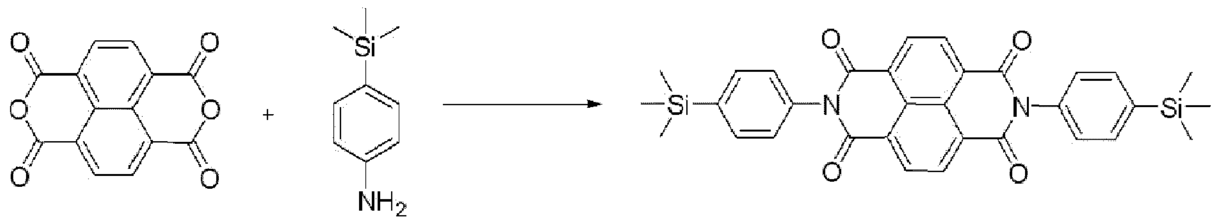

A mixture of naphthalene tetracarboxylic dianhydride (1 eq.) and 4-(trimethylsilyl)aniline (2.2 eq.) is dissolved in dimethylformamide (DMF) solvent to form a solution. The solution is placed in a two-necked round-bottomed flask and stirred at 180 °C for 24 hours. Then, the temperature is lowered to room temperature, methanol is added thereto, the product is precipitated, and the product is filtered to obtain a powder-like material. The material is then washed with methanol several times and purified by recrystallization using ethyl acetate and dimethyl sulfoxide (DMSO). The obtained product is then placed in an oven and dried in vacuum at a temperature of 80 °C for 24 hours to obtain a compound represented by Chemical Formula 1-4 (Compound 1-17 of Group 2). A yield is 28%.

The obtained compound is confirmed through mass and HPLC analysis.

HRMS(MALDI) calcd for C32H30N2O4Si2: m/z: 562.1744, Found: 562.1742

**Comparative Synthesis Example 1-1: Synthesis of Compound Represented by Chemical Formula 1-1C**

[Chemical Formula 1-1C]

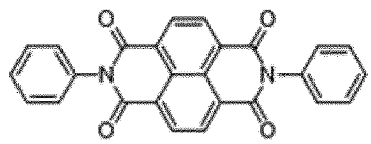

[Reaction Scheme 1-1C]

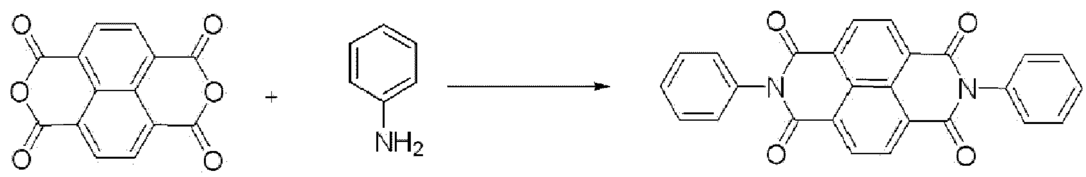

A mixture of naphthalene tetracarboxylic dianhydride (1 eq.) and aniline (2.2 eq.) is dissolved in dimethylformamide (DMF) solvent and placed in a two-necked round-bottomed flask at 180 °C for 24 hours. Then, the temperature is lowered to room temperature, methanol is added thereto, the product is precipitated, and the product is filtered to obtain a powder-like material. The material is then washed with methanol several times and purified by recrystallization

using ethyl acetate and dimethyl sulfoxide (DMSO). The obtained product is then placed in an oven and dried in vacuum at a temperature of 80 °C for 24 hours to obtain a compound represented by Chemical Formula 1 -1C. A yield is 50% or more.

The obtained compound is confirmed through mass and HPLC analysis. HRMS(MALDI) calcd for C26H14N2O4: m/z: 418.0954, Found: 418.0951

**Synthesis Example 2-1: Synthesis of Compound 2-1**

[Compound 2-1]

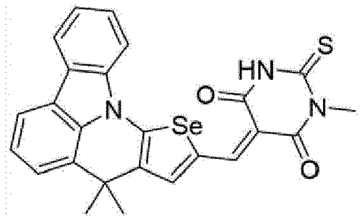

[Reaction Scheme 2-1]

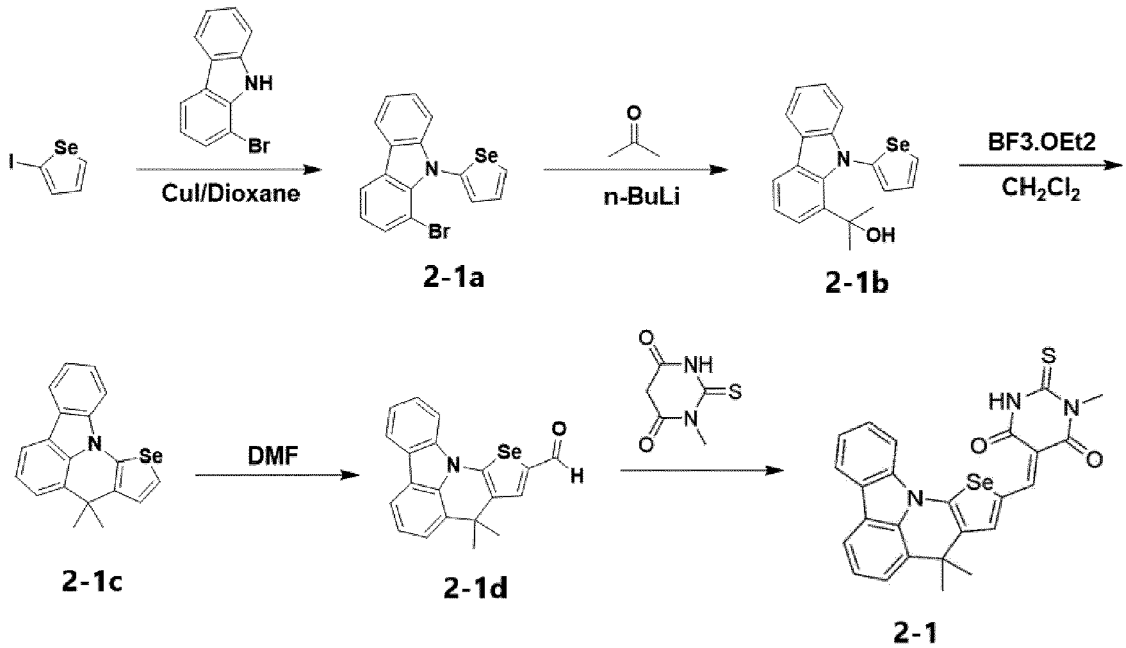


2-1a

2-1b

2-1c

2-1d

2-1

(i) Synthesis of Compound 2-1a

9.4 g (36.5 mmol) of 2-iodoselenophene and 7.5 g (30.5 mmol) of 1-bromo-9H-carbazole are dissolved in 30 ml of dioxane. Subsequently, 0.29 g (1.52 mmol) of copper (I) Iodide, 0.70 g (6.09 mmol) of trans-1,2-cyclohexanediamine, and 12.9g (61.0 mmol) of tripotassium phosphate are added thereto and then, heated under reflux for 30 hours. Herein, a product obtained therefrom (Compound 2-1a) is separated and purified through silica gel column chromatography (a volume ratio of hexane : ethyl acetate = 5 : 1) to obtain 8.18 g (a yield: 72%) of Compound 2-1a.

(ii) Synthesis of Compound 2-1b

12.0 g (32.0 mmol) of Compound 2-1a is dissolved in 300 ml of dehydrated diethyl ether. Subsequently, 12 ml (32.0 mmol) of a 2.76 M n-butyl lithium (n-BuLi) hexane solution is added dropwise thereto at -50 °C and then, stirred

at room temperature for 1 hour. In addition, 2.0 g (35.2 mmol) of dehydrated acetone (dimethylketone ($CH_3COCH_3$)) is added thereto at -50 °C and then, stirred at room temperature for 2 hours. Then, an organic layer extracted from the diethyl ether is washed with a sodium chloride aqueous solution and then, dried by adding anhydrous magnesium sulfate thereto. Herein, a product obtained therefrom (Compound 2-1b) is separated and purified through silica gel column chromatography (by changing the volume ratio of hexane : dichloromethane within a range of 100:0 to 50:50) to obtain 6.3 g (a yield: 56%) of Compound 2-1b.

(iii) Synthesis of Compound 2-1c

6.23 g (17.6 mmol) of Compound 2-1b is dissolved in 180 ml of dichloromethane. Subsequently, 4.98 g (35.5 mmol) of a boron trifluoride-ethyl ether complex ($BF_3 \cdot OEt_2$) is added dropwise thereto at 0 °C and then, stirred for 2 hours. Then, an organic layer extracted from the dichloromethane is washed with a sodium chloride aqueous solution and then, dried by adding anhydrous magnesium sulfate thereto. Herein, a product obtained therefrom (Compound 2-1c) is separated and purified through silica gel column chromatography (in a volume ratio of hexane : dichloromethane = 50 : 50) to obtain 5.12 g (a yield: 87%) of Compound 2-1c. The above process is repeated to obtain the desired amount.

(iv) Synthesis of Compound 2-1d

1.9 ml (20.2 mmol) of phosphoryl chloride is added dropwise to 6.0 ml (77.5 mmol) of N,N-dimethyl formamide (DMF) at -15 °C and then, stirred at room temperature for 2 hours. This solution is slowly added dropwise to 150 ml a dichloromethane solution of 5.23 g (15.5 mmol) of Compound 2-1c at -15 °C and then, concentrated under a low pressure by stirring at room temperature for 30 hours. After adding water thereto, a sodium hydroxide aqueous solution is added thereto until pH becomes 14 and then, stirred at room temperature for 2 hours. Subsequently, an organic layer extracted with the dichloromethane is washed with a sodium chloride aqueous solution and then, dried by adding anhydrous magnesium sulfate thereto. A product obtained therefrom (Compound 2-1d) is separated and purified through silica gel column chromatography (in a volume ratio of hexane : dichloromethane = 50 : 50) to obtain 3.34 g (a yield: 65%) of Compound 2-1d.

(v) Synthesis of Compound 2-1

2.00 g (5.55 mmol) of 4,4-dimethyl-4H-selenopheno[3',2':5,6]pyrido[3,2,1-jk]carbazole-2-carbaldehyde (Compound 2-1d) is suspended in ethanol, 1.05 g (6.66 mmol) of 1-methyl-2-thioxodihydropyrimidine-4,6(1H,5H)-dione is added and then, concentrated under a reduced pressure by stirring at 50 °C for 24 hours to obtain 2.4 g of Compound 2-1. A yield is 86%. The obtained compound is purified by sublimation to purity of 99.9%.

$^1$H-NMR (500 MHz, Methylene Chloride-d2): $\delta$ 8.95 (s, 0.5H), 8.77 (s, 0.5H), 8.65 (s,1H), 8.18 (s, 1H) 8.06 (d, 1H), 7.92 (d, 1H), 7.83 (d, 1H), 7.62 (d, 1H), 7.44 (t, 1H), 7.36 (m, 2H), 3.76 (s, 1.5H), 3.71 (s, 1.5H), 1.68 (s, 6H).

## Examples

### Example 1A

Al (10 nm), ITO (100 nm), and Al (8 nm) are sequentially deposited on a glass substrate to form a first electrode (lower electrode) with an Al/ITO/Al structure (work function: 4.9 eV). Subsequently, N,N'-bis(3-methylphenyl)-N,N'-diphenyl-[1,1-biphenyl]-4,4'-diamine is deposited on the first electrode to form a 170 nm-thick hole auxiliary layer (HOMO: 5.3 to 5.6 eV, LUMO: 2.0 to 2.3 eV). Then, the compound (p-type semiconductor, HOMO: 5.66 eV, LUMO: 3.70 eV) obtained in Synthesis Example 2-1 is deposited at a deposition rate of 0.35 Å/s on the hole auxiliary layer to form a 15 nm-thick p-type layer and then the compound (n-type semiconductor, HOMO: 6.91 eV, LUMO: 3.63 eV) obtained in Synthesis Example 1-1 is deposited at a deposition rate of 0.35 Å/s thereon to form a 35 nm-thick n-type layer, forming a bilayered active layer ($\lambda_{max}$= 556 nm). Then, 4,7-diphenyl-1,10-phenanthroline is deposited on the active layer to form an electron auxiliary layer (HOMO: 6.1 to 6.4 eV, LUMO: 2.9 to 3.2 eV). Then, magnesium and silver are deposited on the electronic auxiliary layer to form a Mg:Ag (in a volume ratio of 1:10) upper electrode to manufacture a photoelectric device.

### Example 1B

A photoelectric device is manufactured in the same manner as Example 1A, except that the compound obtained in Synthesis Example 1-1 (n-type semiconductor, HOMO: 6.91 eV, LUMO: 3.63 eV) is deposited at a deposition rate of 10 Å/s to form a 35 nm-thick n-type layer.

**Example 2A**

A photoelectric device is manufactured in the same manner as Example 1A, except that the compound obtained in Synthesis Example 1-2 is used as an n-type semiconductor instead of the compound obtained in Synthesis Example 1-1.

**Example 2B**

A photoelectric device is manufactured in the same manner as Example 1A, except that the compound obtained in Synthesis Example 1-2 is used as an n-type semiconductor instead of the compound obtained in Synthesis Example 1-1, and the deposition rate when forming the n-type layer is changed to 10 Å/s.

**Comparative Example 1A**

A photoelectric device is manufactured in the same manner as Example 1A, except that the compound obtained in Comparative Synthesis Example 1-1 is used as an n-type semiconductor instead of the compound obtained in Synthesis Example 1-1.

**Comparative Example 1B**

A photoelectric device is manufactured in the same manner as Example 1B, except that the compound obtained in Comparative Synthesis Example 1-1 is used as an n-type semiconductor instead of the compound obtained in Synthesis Example 1-1.

**Evaluation 1: Volume of Compound, Volume of Substituent, Volume Ratio of Substituent, and Energy Disorder**

For the compounds of Group 2, the volume of the compound ($V_a$) is calculated as in Equation 1 under the b3lyp/6-31G condition using Gaussian 09, and the volume of the substituent ($V_a$-$V_{Ref}$) is obtained by subtracting the volume ($V_{Ref}$) calculated under b3lyp/6-31G conditions using Gaussian 09 of the compound represented by Chemical Formula Ref from the volume of the compound ($V_a$). Also, for the compounds of Group 2, the volume ratios of the substituents ($X^1$ and $X^2$) are calculated as in Equation 2. The results are listed in Table 1. For comparison, the volume of the compound, the volume of the substituent, and the volume ratio of the substituent are calculated using the same method for the compounds of Group 2C and are listed in Table 1.

[Equation 1]

$$\text{Volume } (V_b) \text{ of substituent} = V_a\text{-}V_{Ref}$$

[Equation 2]

$$\text{Volume ratio of a substituent} = V_b/V_a$$

In Equations 1 and 2, $V_a$ is a volume of the compound, $V_b$ is a volume of the substituents ($X^1$ and $X^2$), and $V_{Ref}$ is a volume of the compound represented by Chemical Formula Ref'.

[Chemical Formula Ref']

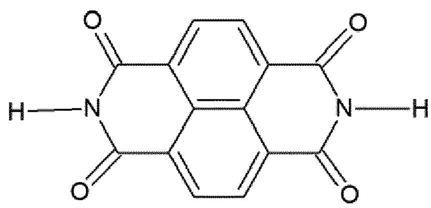

"Energy disorder" is measured by creating a morphology with 600 molecules through molecular dynamics simulation using the Schrodinger company's Desmond software and OPLS4 force field parameters; sampling 200 molecules in this morphology; fitting the HOMO energy distribution obtained through DFT calculations using the Schrodinger company's Jaguar under the b3lyp/LACV3P** conditions to a normal distribution; and determining the width ($\sigma$ = energy disorder) of the distribution. The results are shown in Table 1.

[Group 2C]

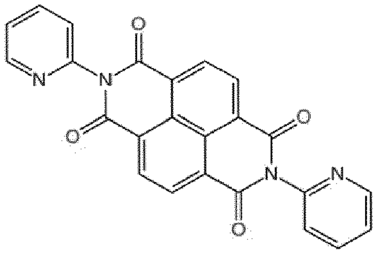

(1-2C)

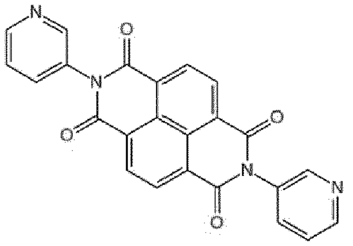

(1-3C)

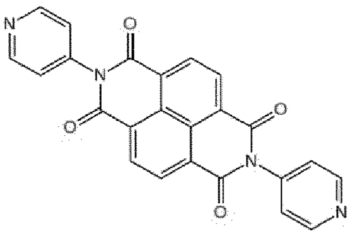

(1-4C)

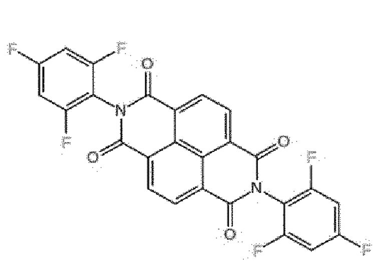

(1-5C)

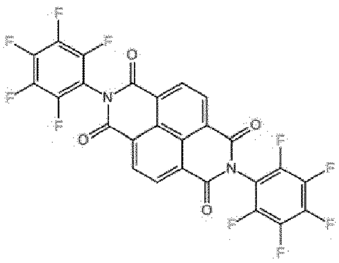

(1-6C)

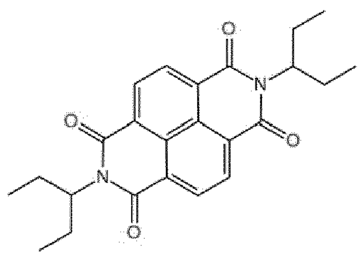

(1-7C)

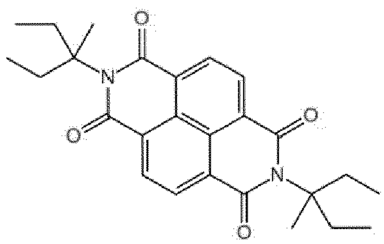

(1-8C)

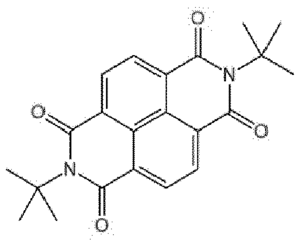

(1-9C)

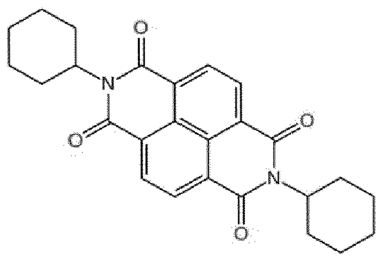

(1-10C)

(Table 1)

| Compound number | Total volume $V_a$ [bohr$^3$] | Volume of substituent ($X^1$ and $X^2$) ($V_b$) [bohr$^3$] | Volume of one substituent ($X^1$ or $X^2$) ($V_b/2$) [bohr$^3$] | Substituent volume ratio ($V_b/V_a$)(%) | Molecular weight (g/mol) | Energy Disorder (eV) |
|---|---|---|---|---|---|---|
| 1-4 | 4170.61 | 2316.34 | 1158.17 | 55.54 | 502.19 | 0.17945 |
| 1-6 | 3877.93 | 2023.66 | 1011.83 | 52.18 | 520.68 | 0.1641 |
| 1-7 | 3939.03 | 2084.76 | 1042.38 | 52.93 | 518.53 | 0.1844 |
| 1-8 | 4053.92 | 2199.65 | 1099.83 | 54.26 | 518.53 | 0.1873 |
| 1-9 | 4349.35 | 2495.08 | 1247.54 | 57.37 | 558.68 | 0.1842 |
| 1-10 | 4426.91 | 2572.64 | 1286.32 | 58.11 | 570.60 | 0.1858 |

(continued)

| Compound number | Total volume $V_a$ [bohr$^3$] | Volume of substituent ($X^1$ and $X^2$) ($V_b$) [bohr$^3$] | Volume of one substituent ($X^1$ or $X^2$) ($V_b/2$) [bohr$^3$] | Substituent volume ratio ($V_b/V_a$)(%) | Molecular weight (g/mol) | Energy Disorder (eV) |
|---|---|---|---|---|---|---|
| 1-11 | 3845.28 | 1991.02 | 995.51 | 51.78 | 570.60 | 0.1965 |
| 1-12 | 4500.42 | 2646.15 | 1323.08 | 58.80 | 570.60 | 0.1487 |
| 1-13 | 5944.14 | 4089.87 | 2044.94 | 68.81 | 722.80 | 0.1445 |
| 1-14 | 5701.62 | 3847.35 | 1923.68 | 67.48 | 722.80 | 0.1545 |
| 1-15 | 4967.68 | 3113.41 | 1556.71 | 62.67 | 618.65 | 0.1902 |
| 1-16 | 4687.27 | 2833.00 | 1416.50 | 60.44 | 618.65 | 0.1952 |
| 1-20 | 5206.04 | 3351.77 | 1675.89 | 64.38 | 651.86 | 0.1818 |
| 1-23 | 6629.54 | 4775.27 | 2387.64 | 72.03 | 835.02 | 0.1420 |
| 1-24 | 4062.90 | 2662.97 | 1331.49 | 65.54 | 582.70 | 0.1804 |
| 1-25 | 4517.24 | 3090.30 | 1545.15 | 68.41 | 582.70 | 0.1835 |
| 1-26 | 4944.57 | 3201.22 | 1600.61 | 64.74 | 582.70 | 0.1810 |
| 1-27 | 5055.49 | 3576.20 | 1788.10 | 70.74 | 670.72 | 0.1593 |
| 1-28 | 5430.47 | 4413.09 | 2206.55 | 81.27 | 953.20 | 0.1346 |
| 1-29 | 6267.35 | 2873.48 | 1436.74 | 45.85 | 598.57 | 0.1830 |
| 1-30 | 4727.75 | 2462.94 | 1231.47 | 52.10 | 630.69 | 0.2146 |
| 1-1C | 2868.22 | 1013.95 | 506.975 | 35.35 | 418.41 | 0.2331 |
| 1-2C | 2841.56 | 987.30 | 493.65 | 34.74 | 420.38 | 0.2673 |
| 1-3C | 3349.50 | 1495.23 | 747.615 | 44.64 | 420.38 | 0.2568 |
| 1-4C | 2988.24 | 1133.97 | 566.985 | 37.95 | 420.38 | 0.2617 |
| 1-5C | 3168.22 | 1313.96 | 656.98 | 41.47 | 526.35 | 0.1277 |
| 1-6C | 3385.79 | 1531.52 | 765.76 | 45.23 | 690.40 | 0.1673 |
| 1-7C | 3267.83 | 1413.56 | 706.78 | 43.26 | 406.48 | 0.1461 |
| 1-8C | 3419.10 | 1564.83 | 782.415 | 45.77 | 434.54 | 0.2159 |
| 1-9C | 2680.22 | 825.96 | 412.98 | 30.82 | 378.43 | 0.2204 |
| 1-10C | 3390.28 | 1536.01 | 768.01 | 45.31 | 430.50 | 0.1492 |

Referring to Table 1, Compounds 1-4 and 1-6 to 1-30 have a range of compounds according to some example embodiments (the volume of compound is about 3800 to about 7000 bohr$^3$, the volume of one substituent ($X^1$ or $X^2$) is about 900 to about 3000 bohr$^3$, and the volume ratio of substituents is within the range of about 45% to about 85%). In comparison, the volumes of Compounds 1-1C to 1-10C are less than 3800 bohr$^3$ and the volumes of one substituent thereof are less than 900 bohr$^3$, and thus they are outside the range of compounds according to some example embodiments.

**Evaluation 2: Energy Level and Energy Bandgap of Compounds**

The energy levels (HOMO, LUMO) of the compounds obtained in the Synthesis Examples are evaluated.

HOMO energy levels are evaluated with an amount of photoelectrons emitted by energy when irradiating UV light to a thin film using AC-2 (Hitachi) or AC-3 (Riken Keiki Co., Ltd.). Energy bandgaps are obtained by using a UV-Vis spectrometer (Shimadzu Corp.). Then, LUMO energy levels are calculated by using the energy bandgaps and the HOMO energy levels. The results are shown in Table 2.

(Table 2)

| | HOMO (eV) | LUMO (eV) | Energy bandgap (eV) |
|---|---|---|---|
| Synthesis Example 1-1 | -6.91 | -3.63 | 3.28 |
| Synthesis Example 1-2 | -6.91 | -3.65 | 3.26 |
| Synthesis Example 1-3 | -6.93 | -3.64 | 3.29 |
| Synthesis Example 1-4 | -7.09 | -3.68 | 3.41 |
| Comparative Synthesis Example 1-1C | -7.22 | -3.70 | 3.52 |

Referring to Table 2, the compounds in Synthesis Example 1-1 to 1-4 can be used as an n-type semiconductor.

**Evaluation 3: Reorganization Energy and Oscillator strength of Compounds**

The reorganization energy and oscillator strength of the compounds according to Synthesis Examples 1-1 to 1-4 and Comparative Synthesis Example 1-1C are obtained by calculating at the DFT B3LYP/DGDZVP level using the Gaussian 09 program. The results are shown in Tables 3 and 4.

(Table 3)

| | Reorganization energy (eV) |
|---|---|
| Synthesis Example 1-1 | 0.373 |
| Synthesis Example 1-2 | 0.365 |
| Synthesis Example 1-3 | 0.363 |
| Synthesis Example 1-4 | 0.382 |

(Table 4)

| | Oscillator Strength (a.u.) |
|---|---|
| Synthesis Example 1-1 | 0.47 |
| Synthesis Example 1-2 | 0.49 |
| Synthesis Example 1-3 | 0.46 |
| Synthesis Example 1-4 | 0.48 |
| Comparative Synthesis Example 1-1C | 0.094 |

Referring to Tables 3 and 4, the reorganization energies of the compounds of Synthesis Examples 1-1 to 1-4 are expected to improve the charge mobility in the active layer of the photoelectric device because the reorganization energies have a low value of 0.382 or less, and it can be predicted that the absorption coefficients are improved because the oscillator strengths of Synthesis Examples 1-1 to 1-4 are high.

**Evaluation 4: Sublimation Temperature of Compound**

The sublimation temperatures of the compounds in the Synthesis Examples are measured.
The sublimation temperature is evaluated through thermogravimetric analysis (TGA) by measuring a temperature where a weight of each sample decreases by 10% from the initial weight by increasing a temperature under a high vacuum (about 10 Pa or less).
The results are shown in Table 5.

(Table 5)

| | $T_{s(10)}$ (°C) |
|---|---|
| Synthesis Example 1-1 | 262 |
| Synthesis Example 1-2 | 244 |
| Synthesis Example 1-3 | 264 |
| Synthesis Example 1-4 | 253 |
| * * $T_{s(10)}$ (°C): A temperature (sublimation temperature) where a weight of each sample decreases by 10% from the initial weight by increasing a temperature under a high vacuum (about 10 Pa or less) | |

Referring to Table 5, the compounds of Synthesis Examples 1-1 to 1-4 exhibit a low sublimation temperature and accordingly, exhibit excellent deposition stability.

**Evaluation 5: Photoelectric Conversion Efficiency of Photoelectric Device**

The photoelectric conversion efficiency of the photoelectric devices according to Example 1A, Example 1B, Example 2A, Example 2B, Comparative Example 1A, and Comparative Example 1B is evaluated.

The photoelectric conversion efficiency is evaluated from the external quantum efficiency (EQE) measured after the photoelectric devices according to Example 1A, Example 1B, Example 2A, Example 2B, Comparative Example 1A and Comparative Example 1B are left at 85 °C for 1 hour. The external quantum efficiency (EQE) is evaluated by using incident photon to current efficiency (IPCE) at a wavelength of 450 nm (blue, B), 530 nm (green, G), and 630 nm (red, R).

The results are shown in Table 6.

(Table 6)

| | Relative ratio of EQE(G) (3V, %) | | EQE(G) change ratio |
|---|---|---|---|
| | EQE(G)/EQE (B) | EQE(G)/EQE(R) | |
| Example 1A | 21.19 | 1335.00 | |
| Example 1B | 28.14 | 1365.00 | 2.2% increase compared to Example 1A |
| Example 2A | 15.96 | 1420.00 | |
| Example 2B | 18.66 | 2108.50 | 1.0% decrease compared to Example 2A |
| Comparative Example 1A | 92.97 | 526.83 | |
| Comparative Example 1B | 23.33 | 462.00 | 26.9% decrease compared to Comparative Example 1A |

Referring to Table 6, the photoelectric devices according to Example 1A, Example 1B, Example 2A, and Example 2B have higher EQE(G) in the green wavelength region than EQE(B) and EQE(R) in the blue and red wavelength regions, indicating that selectivity in the green wavelength region is improved. In addition, the EQE of the photoelectric devices according to Examples 1B and 2B shows almost no change in EQE even when the deposition rate is increased, whereas the EQE of the photoelectric devices according to Comparative Example 1B decreases significantly.

**Evaluation 6: Dark Current of Photoelectric Device**

The dark currents of the photoelectric devices according to Example 1B, Example 2B, and Comparative Example 1B are evaluated at room temperature and high temperature (after being left at 85° C. for 1 hour) under a reverse bias voltage.

The dark current is evaluated with dark current density, which is obtained by using a current-voltage evaluating equipment (Keithley K4200 parameter analyzer) and dividing it by a unit pixel area (0.04 $cm^2$), and the dark current density is evaluated from a current flowing when a reverse bias of -3 V is applied thereto.

The results are shown in Table 7.

(Table 7)

| | Dark current density (mA/cm$^2$) | |
|---|---|---|
| | Room temperature | High temperature |
| Example 1B | $0.87 \times 10^{-6}$ | $1.5 \times 10^{-6}$ |
| Example 2B | $2.2 \times 10^{-6}$ | $2.0 \times 10^{-6}$ |
| Comparative Example 1B | $5.6 \times 10^{-6}$ | $6.6 \times 10^{-6}$ |

Referring to Table 7, the dark current density of the photoelectric devices according to Example 1B and Example 2B is reduced compared to the photoelectric device according to Comparative Example 1B at room temperature and high temperature.

While the present inventive concepts have been described in connection with what is presently considered to be practical example embodiments, it is to be understood that the inventive concepts are not limited to such example embodiments. On the contrary, the scope of the inventive concepts is intended to cover various modifications and equivalent arrangements included within the scope of the appended claims.

**<Description of symbols>**

| | | | | | |
|---|---|---|---|---|---|
| 10: | first electrode | 20: | second electrode | | |
| 30: | active layer | 40, 45: | charge auxiliary layer | | |
| 100, 200, 100a, 100b, 100c, 100aa, 100bb, 100cc, 100dd: | photoelectric device | | | | |
| 300, 400, 500, 600, 700, 800: | organic CMOS image sensor | | | | |
| 310: | semiconductor substrate | 70B, 72B: | blue filter | 70R, 72R: | red filter |
| 70, 72: | color filter layer | 85, 85a, 85b, 85c: | through-hole | | |
| 60: | lower insulation layer | 80: | upper insulation layer | | |
| 50B, 50R: | photo-sensing device | 55: | charge storage | | |
| 1000: | digital camera | 1010: | lens | | |
| 1020: | image sensor | 1030: | motor | | |
| 1040: | engine | 1050: | host/application | | |

## Claims

**1.** A compound represented by Chemical Formula 1:

[Chemical Formula 1]

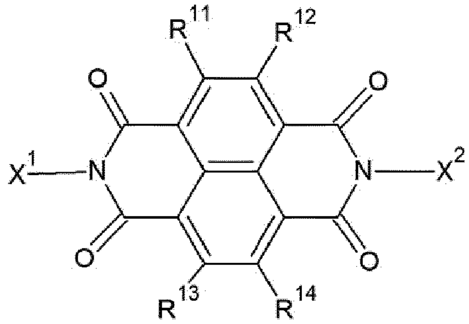

wherein, in Chemical Formula 1,

$X^1$ and $X^2$ are each independently hydrogen, deuterium, tritium, halogen, a cyano group, a substituted or unsubstituted C1 to C10 alkyl group, a substituted or unsubstituted C6 to C30 aryl group, or a substituted or unsubstituted C3 to C30 heteroaryl group,
at least one of $X^1$ or $X^2$ is a substituent having a volume of 900 $bohr^3$ to 3000 $bohr^3$ (V/molecule), and the at least one of $X^1$ or $X^2$ is a substituted or unsubstituted C6 to C30 aryl group or a substituted or unsubstituted C3 to C30 heteroaryl group, and
$R^{11}$, $R^{12}$, $R^{13}$, and $R^{14}$ are each independently hydrogen, deuterium, tritium, a halogen, a cyano group, a C1 to C10 alkyl group, a C1 to C10 alkoxy group, or a C6 to C10 aryl group.

**2.** The compound of claim 1, wherein the compound has an energy disorder of less than or equal to 0.22 eV; and/or wherein the compound has a volume of 3800 $bohr^3$ to 7000 $bohr^3$ (V/molecule).

**3.** The compound of claims 1 or 2, wherein

the compound has a volume ratio of substituents $X^1$ and $X^2$ obtained according to Equation 1 and Equation 2, the volume ratio ranging from 45% to 85%:

[Equation 1]

$$\text{Volume } (V_b) \text{ of substituent} = V_a - V_{Ref}$$

[Equation 2]

$$\text{volume ratio of a substituent} = V_b/V_a$$

wherein, in Equations 1 and 2,
$V_a$ is a volume of the compound, $V_b$ is a volume of the substituents $X^1$ and $X^2$, and $V_{Ref}$ is a volume of a compound represented by Chemical Formula Ref:

[Chemical Formula Ref]

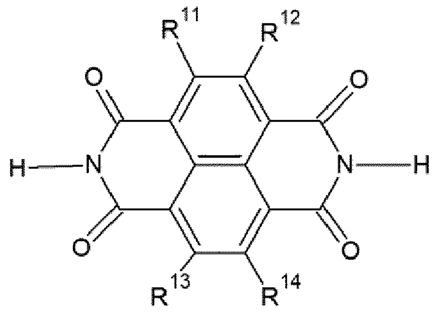

wherein, in Chemical Formula Ref, $R^{11}$, $R^{12}$, $R^{13}$, and $R^{14}$ are the same as in Chemical Formula 1.

**4.** The compound of any of claims 1-3, wherein in Chemical Formula 1, at least one of $X^1$ or $X^2$ includes an electron donating group;
preferably wherein the electron donating group is a C3 to C20 branched alkyl group, a C3 to C20 branched alkoxy group, a C6 to C20 aryl group, a C3 to C20 cycloalkyl group, a C3 to C20 heteroaryl group, a C3 to C20 heterocycloalkyl group, or any combination thereof.

**5.** The compound of any of claims 1-4, wherein
in Chemical Formula 1, at least one of $X^1$ or $X^2$ includes -Si($R^a$)($R^b$)($R^c$), wherein $R^a$, $R^b$, and $R^c$ are each independently hydrogen, deuterium, tritium, a halogen, a cyano group, a linear or branched C1 to C10 alkyl group, or a C6 to C10 aryl group; -Ge($R^a$)($R^b$)($R^c$), wherein $R^a$, $R^b$, and $R^c$ are each independently hydrogen, deuterium, tritium, a halogen, a cyano group, a linear or branched C1 to C10 alkyl group, or a C6 to C10 aryl group; or any combination thereof.

**6.** The compound of any of claims 1-5, wherein
in Chemical Formula 1, 10% to 100% of hydrogen atoms of at least one of $X^1$ or $X^2$ is substituted with deuterium or tritium based on a total number of hydrogen atoms present in the at least one of $X^1$ or $X^2$.

**7.** The compound of any of claims 1-6, wherein
in Chemical Formula 1, at least one of $X^1$ or $X^2$ is a substituent selected from Group 1:

[Group 1]

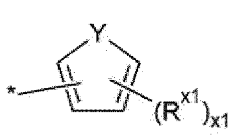
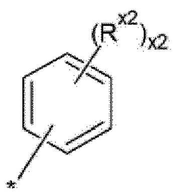
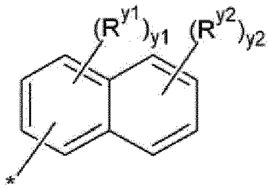
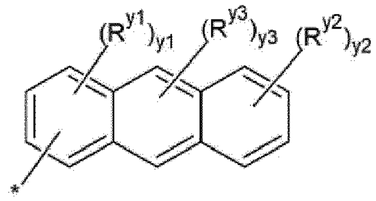
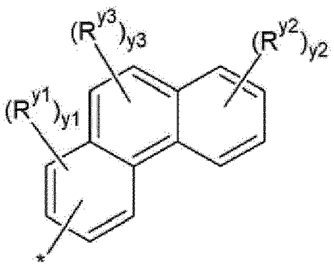
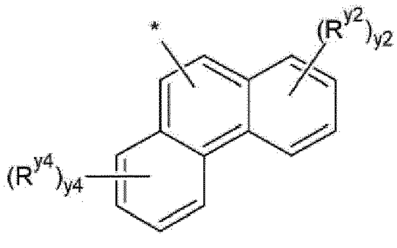
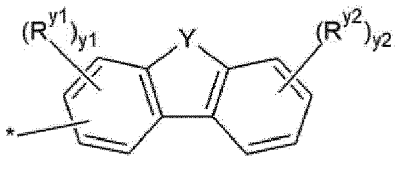

wherein, in Group 1,

Y is S, O, Se, Te, or CRR', wherein R and R' are each independently hydrogen, deuterium, tritium, a C1 to C10 alkyl group, a C1 to C10 alkoxy group, or a C6 to C10 aryl group,
$R^{x1}$ is deuterium, tritium, a C3 to C20 branched alkyl group, a C3 to C20 branched alkoxy group, a C6 to C20 aryl group, a C3 to C20 cycloalkyl group, a C3 to C20 heteroaryl group, a C3 to C20 heterocycloalkyl group, -Si($R^a$)($R^b$)($R^c$), wherein $R^a$, $R^b$, and $R^c$ are each independently hydrogen, deuterium, tritium, a halogen, a cyano group, a linear or branched C1 to C10 alkyl group, or a C6 to C10 aryl group, -Ge($R^a$)($R^b$)($R^c$), wherein $R^a$, $R^b$, and $R^c$ are each independently hydrogen, deuterium, tritium, a halogen, a cyano group, a linear or branched C1 to C10 alkyl group, or a C6 to C10 aryl group, or any combination thereof,
x1 is an integer of 1 to 3,
$R^{x2}$ is deuterium, tritium, a C3 to C20 branched alkyl group, a C3 to C20 branched alkoxy group, a C6 to C20 aryl group, a C3 to C20 cycloalkyl group, a C3 to C20 heteroaryl group, a C3 to C20 heterocycloalkyl group, -Si($R^a$)($R^b$)($R^c$), wherein $R^a$, $R^b$, and $R^c$ are each independently hydrogen, deuterium, tritium, a halogen, a cyano group, a linear or branched C1 to C10 alkyl group, or a C6 to C10 aryl group), -Ge($R^a$)($R^b$)($R^c$), wherein $R^a$, $R^b$, and $R^c$ are each independently hydrogen, deuterium, tritium, a halogen, a cyano group, a linear or branched C1 to C10 alkyl group, or a C6 to C10 aryl group), or any combination thereof,
x2 is an integer of 1 to 5,
$R^{y1}$, $R^{y2}$, $R^{y3}$, and $R^{y4}$ are each independently hydrogen, deuterium, tritium, halogen, a cyano group, C1 to C10 linear alkyl group, C1 to C10 linear alkoxy group, C3 to C20 branched alkyl group, C3 to C20 branched alkoxy group, C6 to C20 aryl group, C3 to C20 cycloalkyl group, C3 to C20 heteroaryl group, C3 to C20 heterocycloalkyl group, -Si($R^a$)($R^b$)($R^c$), wherein $R^a$, $R^b$, and $R^c$ are each independently hydrogen, deuterium, tritium, a halogen, a cyano group, a linear or branched C1 to C10 alkyl group, or a C6 to C10 aryl group), -Ge($R^a$)($R^b$)($R^c$), wherein $R^a$, $R^b$, and $R^c$ are each independently hydrogen, deuterium, tritium, a halogen, a cyano group, a linear or branched C1 to C10 alkyl group, or a C6 to C10 aryl group, or any combination thereof,
y1 is an integer of 0 to 3,
y2 is an integer of 0 to 4,
y3 is an integer of 0 to 2,
y4 is an integer of 0 to 4, and
* is a linking point with Chemical Formula 1.

**8.** The compound of claim 7, wherein
in Group 1, $R^{y1}$, $R^{y2}$, $R^{y3}$, and $R^{y4}$ are each independently deuterium, tritium, halogen, a cyano group, C1 to C10 linear alkyl group, C1 to C10 linear alkoxy group, C3 to C20 branched alkyl group, C3 to C20 branched alkoxy group, C6 to C20 aryl group, C3 to C20 cycloalkyl group, C3 to C20 heteroaryl group, C3 to C20 heterocycloalkyl group, -Si($R^a$)($R^b$)($R^c$), wherein $R^a$, $R^b$, and $R^c$ are each independently hydrogen, deuterium, tritium, a halogen, a cyano group, a linear or branched C1 to C10 alkyl group, or a C6 to C10 aryl group, -Ge($R^a$)($R^b$)($R^c$), wherein $R^a$, $R^b$, and $R^c$ are each independently hydrogen, deuterium, tritium, a halogen, a cyano group, a linear or branched C1 to C10 alkyl group, or a C6 to C10 aryl group, or any combination thereof.

**9.** The compound of any of claims 1-8, wherein the compound has a reorganization energy of less than or equal to 0.390 eV; and/or

wherein the compound has an oscillator strength of greater than or equal to 0.40; and/or
wherein the compound has a molecular weight of less than or equal to 1500 g/mol; and/or
wherein the compound has a sublimation temperature of less than or equal to 340 °C, wherein the sublimation temperature is a temperature at which a 10% weight loss of the compound occurs compared to an initial weight of the compound when thermogravimetrically analyzed at 10 Pa or less.

**10.** A photoelectric device, comprising:

a first electrode and a second electrode facing each other, and
an active layer between the first electrode and the second electrode,
wherein the active layer includes the compound represented by Chemical Formula 1 of any of claims 1-9.

**11.** An image sensor comprising the photoelectric device of claim 10.

**12.** The image sensor of claim 11, further comprising:

a semiconductor substrate integrated with

a plurality of first photo-sensing devices configured to sense light in a blue wavelength region, and
a plurality of second photo-sensing devices configured to sense light in a red wavelength region,

wherein the photoelectric device is on the semiconductor substrate and is configured to selectively sense light in a green wavelength region.

**13.** The image sensor of claims 11 or 12, further comprising a stack of

a green photoelectric device that is configured to selectively sense light in a green wavelength region, wherein the green photoelectric device is the photoelectric device,
a blue photoelectric device configured to selectively sense light in a blue wavelength region, and
a red photoelectric device configured to selectively sense light in a red wavelength region.

**14.** The image sensor of any of claims 11-13, further comprising:

a green photoelectric device that is configured to selectively sense light in a green wavelength region, wherein the green photoelectric device is the photoelectric device,
a blue photoelectric device configured to selectively sense light in a blue wavelength region, and
a red photoelectric device configured to selectively sense light in a red wavelength region,
wherein the green photoelectric device, the blue photoelectric device, and the red photoelectric device are aligned in parallel on a semiconductor substrate and overlap in a horizontal direction that is parallel to an upper surface of the semiconductor substrate.

**15.** An electronic device comprising the image sensor of any of claims 11-14.

FIG. 1

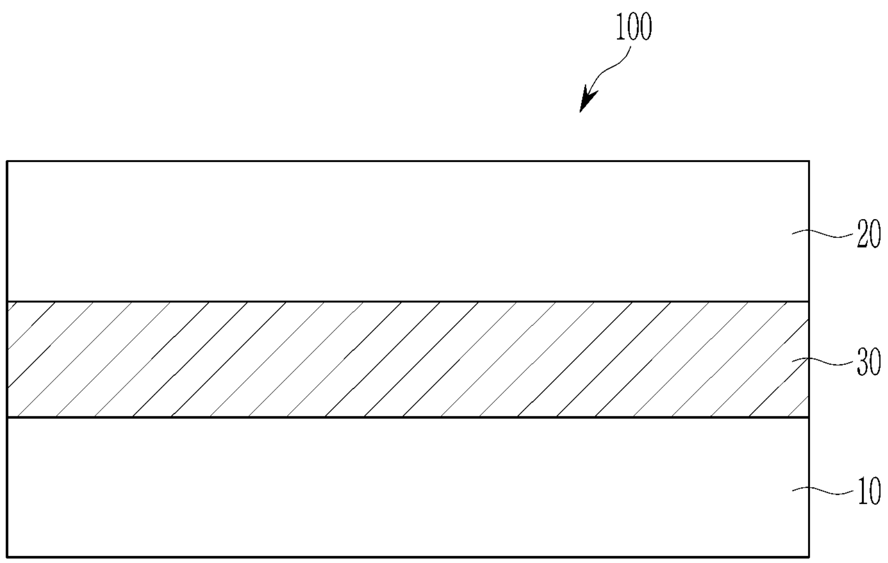

FIG. 2

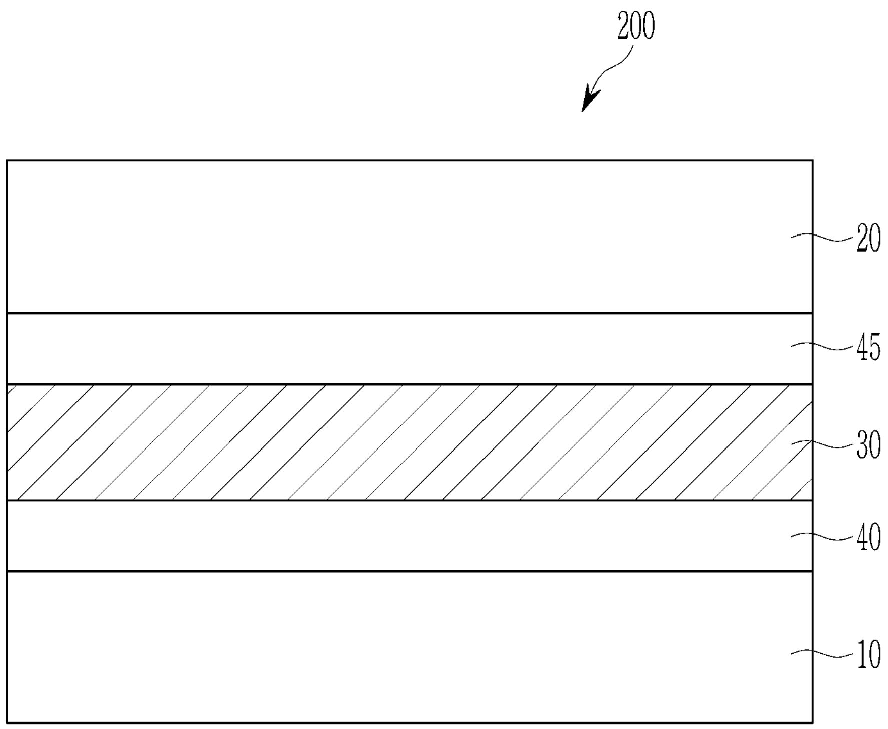

FIG. 3

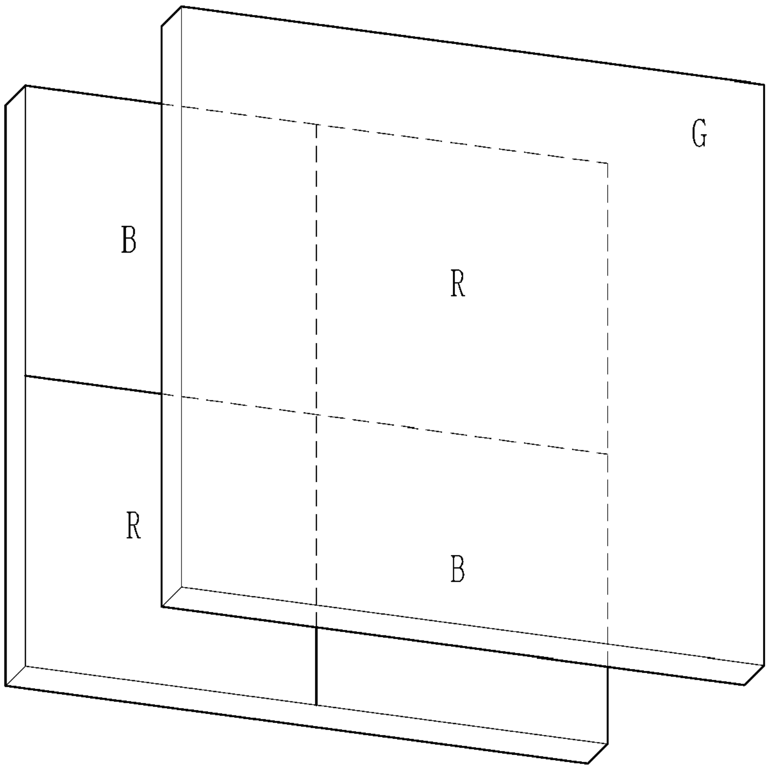

FIG. 4

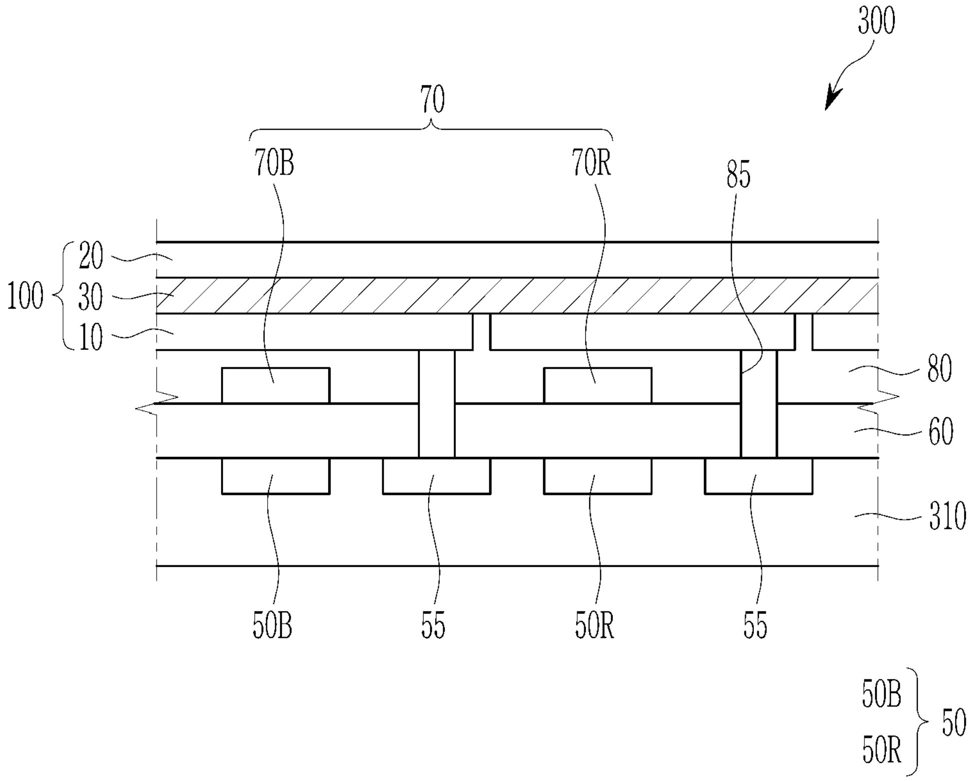

FIG. 5

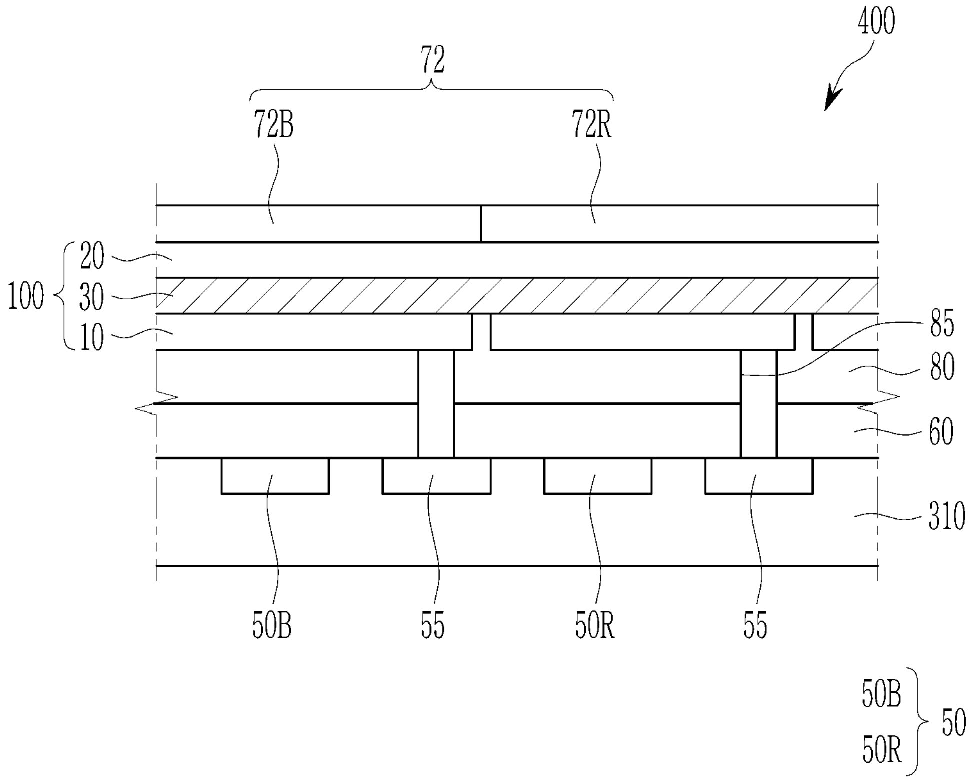

FIG. 6

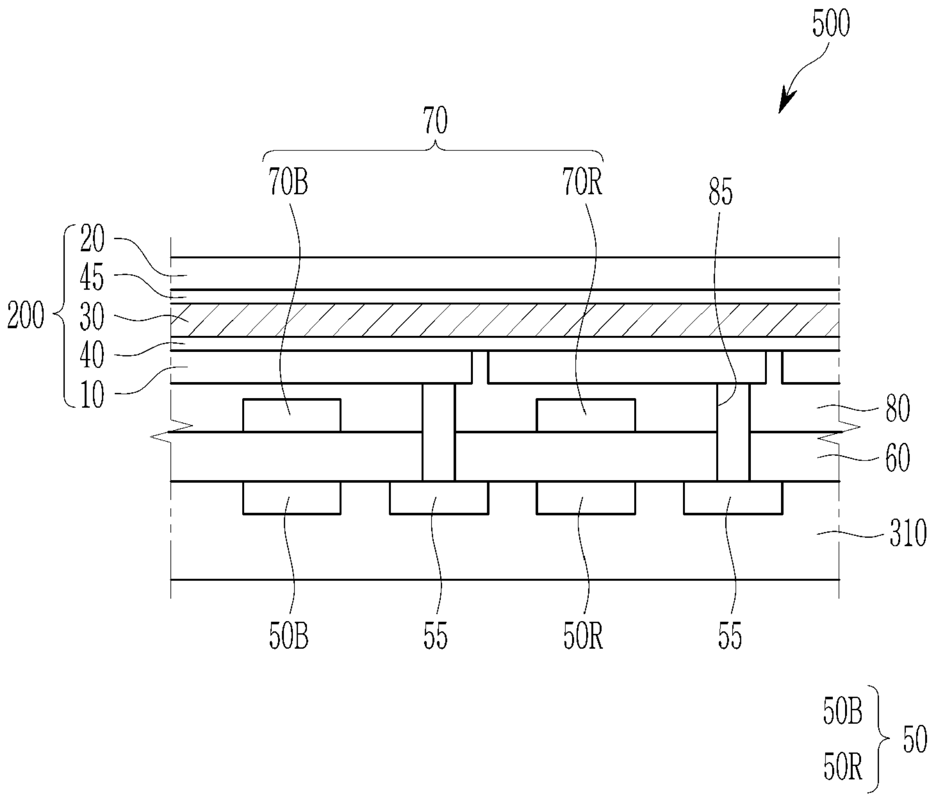
500
70
70B
70R
85
200
20
45
30
40
10
80
60
310
50B
55
50R
55
50B
50R
50

FIG. 7

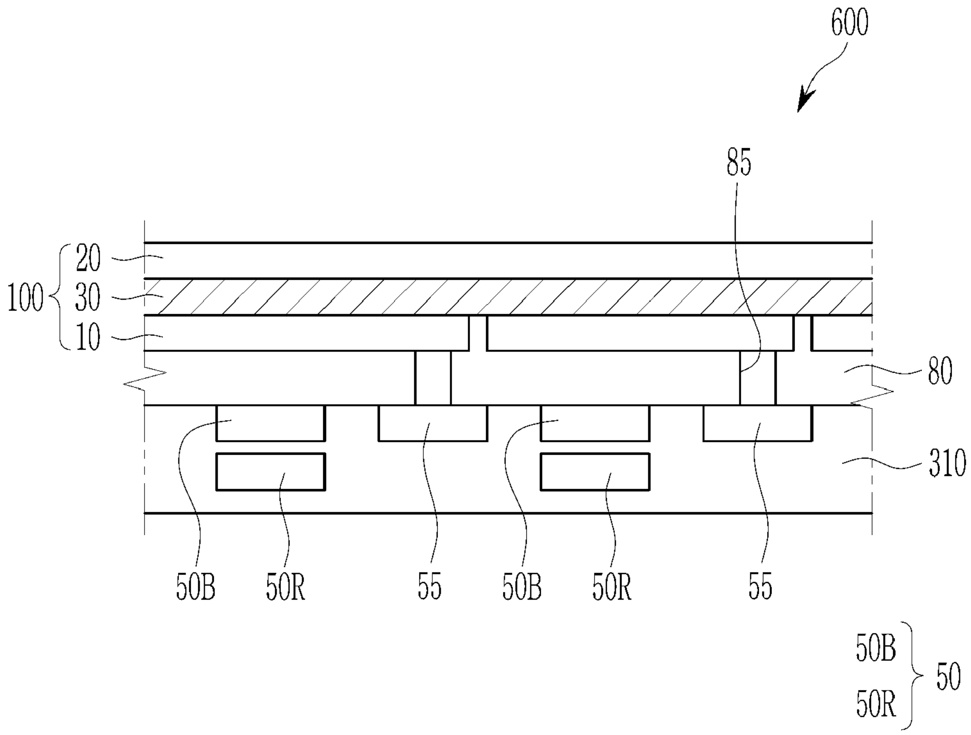

FIG. 8

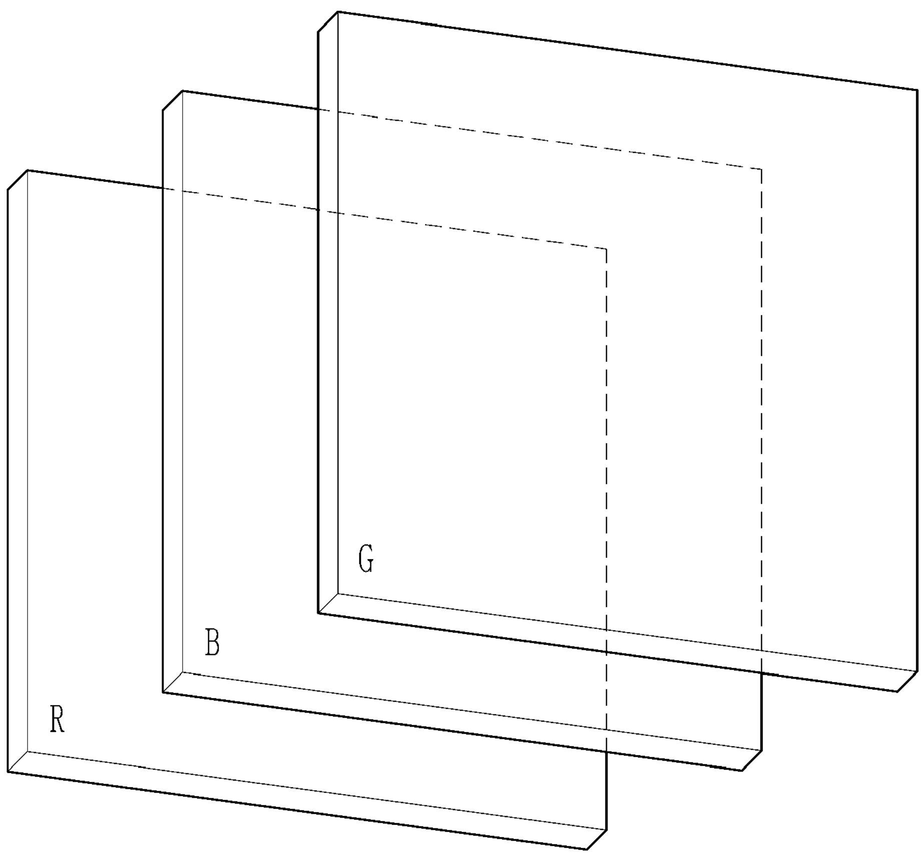

FIG. 9

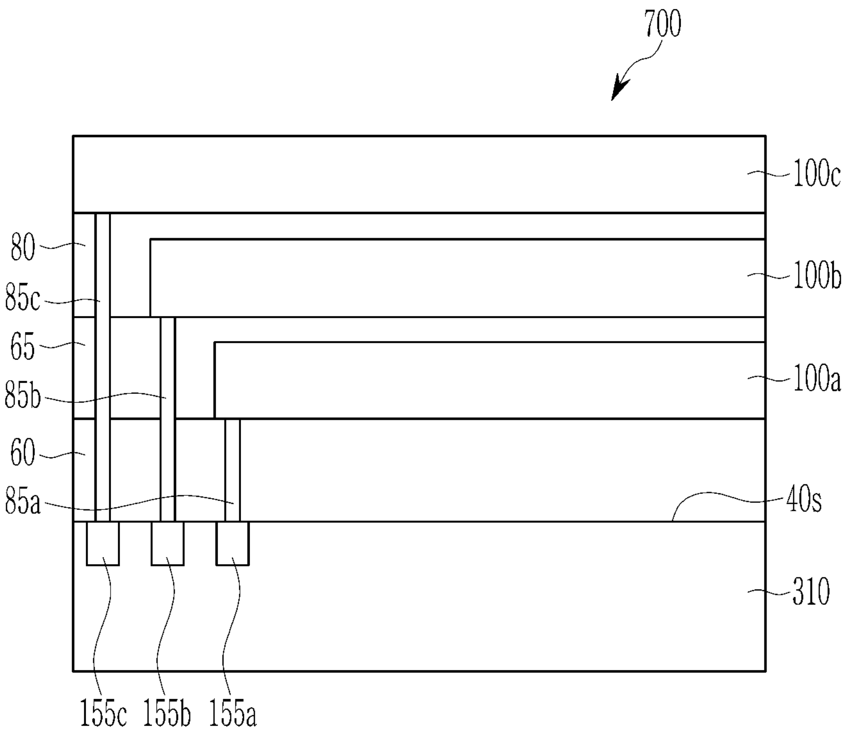

FIG. 10

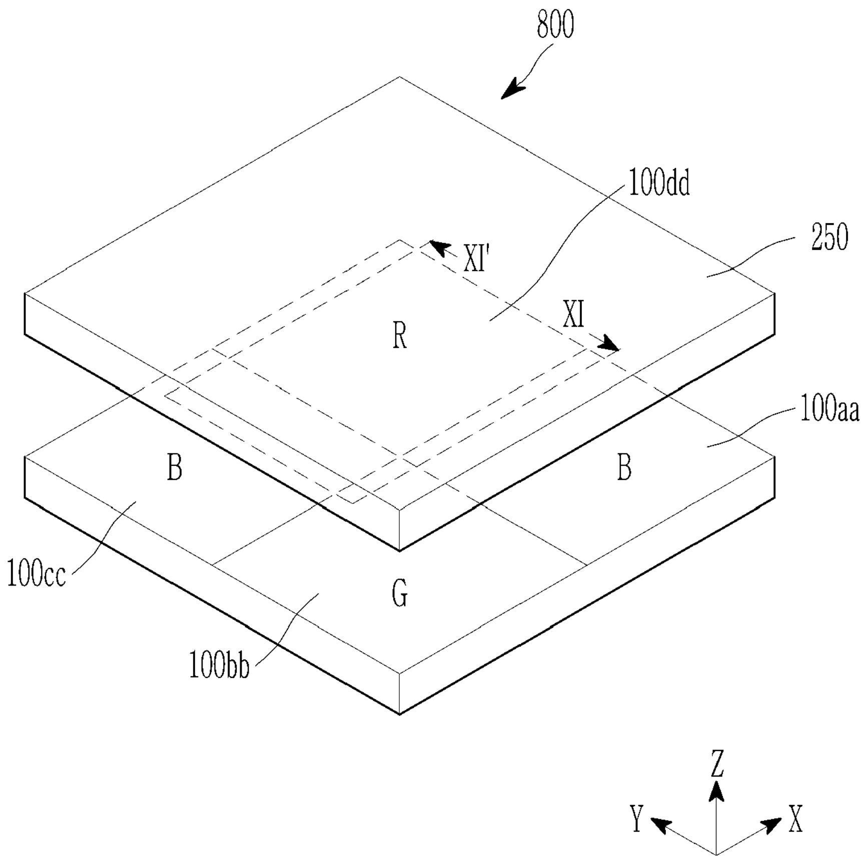

FIG. 11

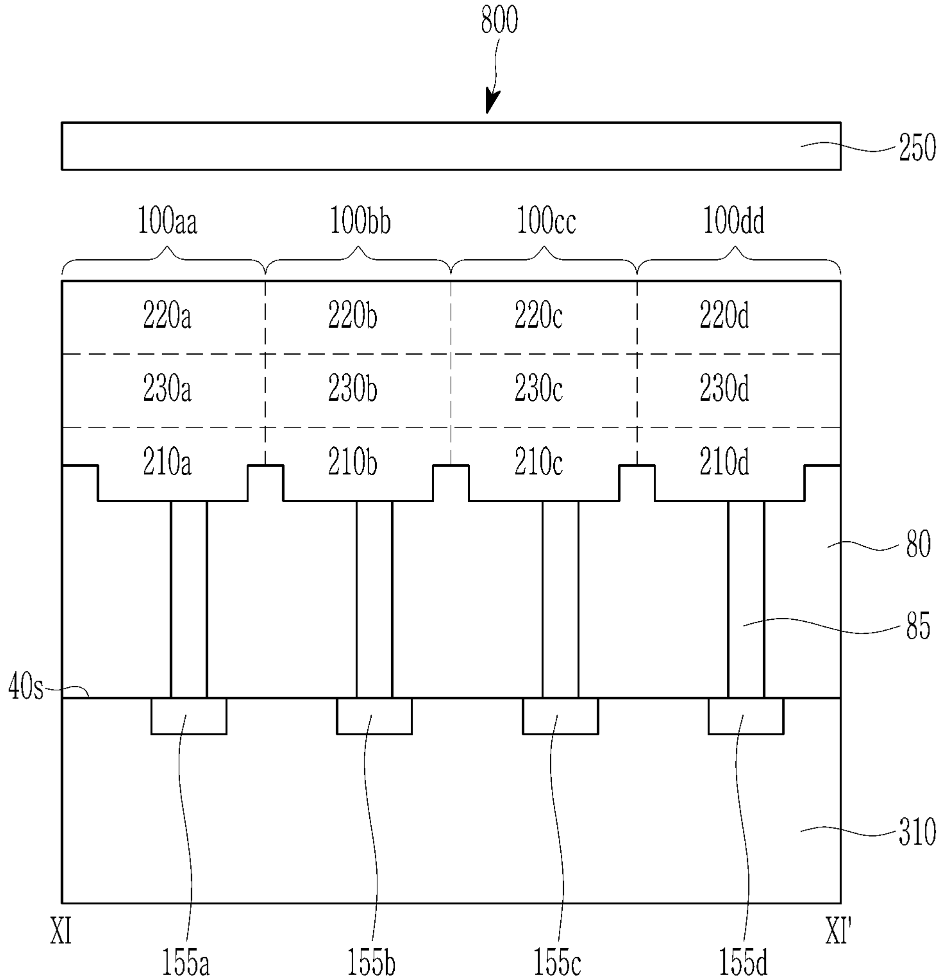
800
250
100aa
100bb
100cc
100dd
220a
220b
220c
220d
230a
230b
230c
230d
210a
210b
210c
210d
80
85
40s
310
XI
XI'
155a
155b
155c
155d

FIG. 12

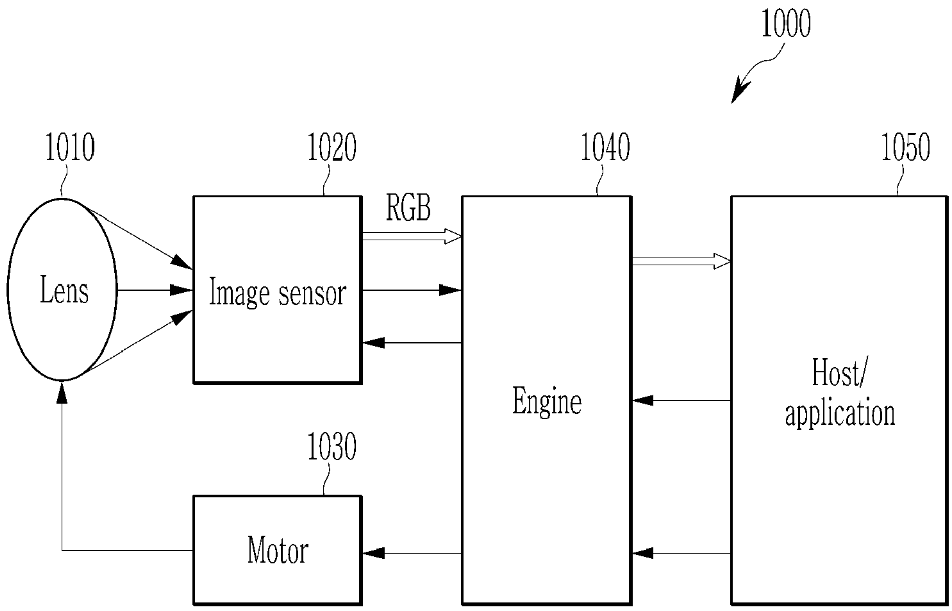
1000
1010
1020
1040
1050
Lens
Image sensor
RGB
Engine
Host/
application
1030
Motor

FIG. 13